# EUROPEAN PATENT APPLICATION

(11) **EP 3 121 163 A1**
(43) Date of publication of application: **25.01.2017**
(21) Application number: 15765128.2
(22) Date of filing: 13.03.2015
(51) Int. Cl.: C07C 233/80, C07C 231/02, C08G 73/00, H01L 51/50

(54) **OLIGOANILINE DERIVATIVE, CHARGE-TRANSPORTING VARNISH, AND ORGANIC ELECTROLUMINESCENT ELEMENT**

(30) Priority: 17.03.2014 JP 2014053431
(71) Applicant: Nissan Chemical Industries, Ltd., Tokyo 101-0054 (JP)
(72) Inventor: NAKAIE, Naoki, Funabashi-shi Chiba 274-0052 (JP)
(74) Representative: Bailey, Sam Rogerson
(86) International application number: PCT/JP2015/057505
(87) International publication number: WO 2015/141585

(57) **Abstract**

Provided are an oligoaniline derivative represented by formula (1), a charge-transporting varnish comprising the oligoaniline derivative, and an organic EL element. 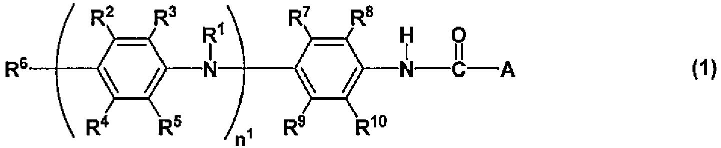 (In the formula, R¹ is a hydrogen atom or optionally substituted alkyl group, R² to R¹⁰ are each independently a hydrogen atom, halogen atom, nitro group, cyano group, or optionally substituted alkyl group, alkenyl group, alkynyl group, aryl group, or heteroaryl group; A is an optionally substituted fluoroalkyl group, fluorocycloalkyl group, fluorobicycloalkyl group, fluoroalkenyl group, or fluoroalkynyl group, optionally substituted fluoroaryl group, substituted phenyl group, optionally substituted fluoroaralkyl group, or substituted aralkyl group; and n¹ is an integer of 1 to 20.)

## Description

### TECHNICAL FIELD

The present invention relates to oligoaniline derivatives, charge-transporting varnishes, and organic electroluminescent (EL) elements.

### BACKGROUND ART

In organic EL elements, a charge-transporting thin film composed of an organic compound is used as a light-emitting layer or a charge injection layer. Particularly, a hole injection layer serves for transfer of electric charges between an anode and a hole transport layer or a light-emitting layer, thereby fulfilling an important function for achieving low-voltage driving and high luminance in the organic EL element.

Methods for forming a hole injection layer are generally classified into dry process typified by the vapor deposition method and wet process typified by the spin coating method. When these processes are compared, the wet process permits more efficient production of thin films with high flatness over a wider area. Under the present trend toward organic EL displays of larger areas, therefore, there is a request for a hole injection layer that can be formed by a wet process.

In consideration of the above-mentioned circumstances, the present inventors have developed charge-transporting materials which are applicable to various wet processes and which give thin films capable of realizing excellent EL element characteristics when applied to the hole injection layer in organic EL elements, and compounds which are to be used for the charge-transporting materials and show good solubility in organic solvents (see, for example, Patent Documents 1 to 4).

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777

### SUMMARY OF THE INVENTION

### PROBLEMS TO BE SOLVED BY THE INVENTION

Like the technologies of the above-mentioned Patent Documents that the present inventors have developed, the present invention has an object to provide novel oligoaniline derivatives which show good solubility in organic solvents and can realize organic EL elements with excellent electrical characteristics when applied to a hole injection layer in the form of a thin film, to provide charge-transporting varnishes containing the oligoaniline derivatives, and to provide organic EL elements.

### MEANS FOR SOLVING THE PROBLEMS

During the developments that have been made by the present inventors, in the case where a fluorine-atom-free compound was used as the charge-transporting substance, a lowering in the characteristics might occur depending on the dopant used together. In order to solve this problem, the present inventors made extensive and intensive investigations. As a result of the investigations, the inventors have found out that specific fluorine-containing oligoaniline derivatives show good solubility in organic solvents, that thin films obtained from varnishes prepared by dissolving the oligoaniline derivatives in organic solvents together with an arbitrary dopant have high charge-transporting properties, and that excellent electrical characteristics can be realized when the thin films are applied to a hole injection layer in organic EL elements. Based on the findings, the inventors have completed the present invention.

Specifically, the present invention provides the following oligoaniline derivatives, charge-transporting varnishes, and organic EL elements.
1. An oligoaniline derivative represented by formula (1): 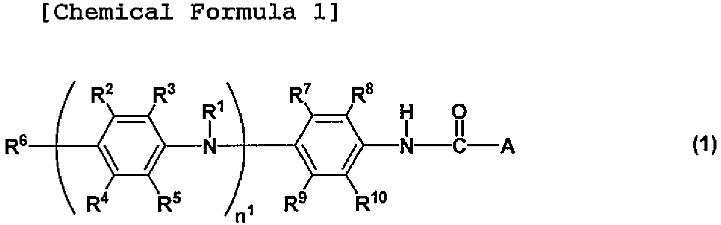 (In the formula, R¹ represents a hydrogen atom or a C₁-C₂₀ alkyl group that may be substituted with Z, Z represents a halogen atom, a nitro group, a cyano group, an aldehyde group, a hydroxyl group, a thiol group, a sulfonic acid group, a carboxylic acid group, a C₆-C₂₀ aryl group that may be substituted with Z', or a C₂-C₂₀ heteroaryl group that may be substituted with Z', and Z' represents a halogen atom, a nitro group, a cyano group, an aldehyde group, a hydroxyl group, a thiol group, a sulfonic acid group, or a carboxylic acid group;
   R² to R¹⁰ each independently represent a hydrogen atom, a halogen atom, a nitro group, a cyano group, or a C₁-C₂ alkyl group, a C₂-C₂₀ alkenyl group, a C₂-C₂₀ alkynyl group, a C₆-C₂₀ aryl group, or a C₂-C₂₀ heteroaryl group that may be substituted with a halogen atom;
   A represents
   a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group that may be substituted with a cyano group, a chlorine atom, a bromine atom, an iodine atom, a nitro group, or a C₁-C₂₀ fluoroalkoxy group,
   a C₆-C₂₀ fluoroaryl group that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group,
   a C₆-C₂₀ aryl group that is substituted with C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group,
   a C₇-C₂₀ fluoroaralkyl group that may be substituted with a cyano group, a chlorine atom, a bromine atom, an iodine atom, a nitro group, a C₁-C₂₀ fluoroalkoxy group, a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group, or
   a C₇-C₂₀ aralkyl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group; and
   letter n¹ represents an integer of 1 to 20.)
2. The oligoaniline derivative of 1 above, wherein A is a C₁-C₂₀ fluoroalkyl group that may be substituted with a cyano group, a chlorine atom, a bromine atom, an iodine atom, a nitro group, or a C₁-C₂₀ fluoroalkoxy group, a C₆-C₂₀ fluoroaryl group that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group, or a C₆-C₂₀ aryl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group.
3. The aniline derivative of 2 above, wherein A is a phenyl group that is substituted with at least three fluorine atoms and that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group, or a phenyl group that is substituted with C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group.
4. The oligoaniline derivative of any one of 1 to 3 above, wherein R¹ is a hydrogen atom.
5. The oligoaniline derivative of any one of 1 to 4 above, wherein R² to R¹⁰ are each a hydrogen atom.
6. The oligoaniline derivative of any one of 1 to 5 above, wherein letter n¹ is 2 to 10.
7. A charge-transporting substance consisting of the oligoaniline derivative of any one of 1 to 6 above.
8. A charge-transporting varnish containing the charge-transporting substance of 7 above and an organic solvent.
9. The charge-transporting varnish of 8 above, wherein the varnish further contains a dopant.
10. A charge-transporting thin film produced by use of the charge-transporting varnish of 8 or 9 above.
11. An electronic device includes the charge-transporting thin film of 10 above.
12. An organic electroluminescent element includes the charge-transporting thin film of 10 above.
13. A method of producing the oligoaniline derivative of 1 above, the method includes reacting an amine compound represented by formula (2) with a fluorine-containing acid halide represented by formula (3). 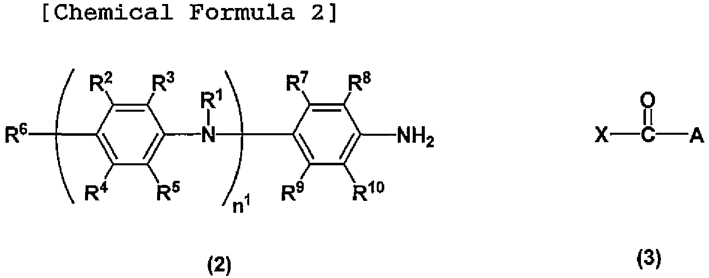
(In the formulas, R¹ to R¹⁰, A, and letter n¹ are the same as defined above, and X represents a halogen atom.)

### ADVANTAGEOUS EFFECTS OF THE INVENTION

Since the oligoaniline derivatives of the present invention contains a fluorine atom, polarity is generated in the molecules of the oligoaniline derivatives, whereby the solubility of the oligoaniline derivatives in organic solvents is enhanced. Therefore, it is easy to prepare charge-transporting varnishes by dissolving the oligoaniline derivatives in an organic solvent together with a dopant.

Since the thin films produced from the
charge-transporting varnishes of the present invention contain the oligoaniline derivatives, the thin films show high charge-transporting properties, irrespectively of the kind of the dopant. Therefore, the thin films can be preferably used as a thin film in electronic devices such as organic EL elements. Particularly, by applying the thin films to a hole injection layer in organic EL elements, it is possible to obtain organic EL elements having excellent luminance characteristics.

In addition, the charge-transporting varnishes of the present invention enable a thin film excellent in charge-transporting properties to be produced with good reproducibility, even when wet processes capable of forming a film over a large area, such as the spin coating method and the slit coating method, are used. Therefore, the charge-transporting varnishes make it possible to
sufficiently cope with the progress in the field of organic EL elements in recent years.

### EMBODIMENT FOR CARRYING OUT THE INVENTION

### [Oligoaniline Derivatives]

The oligoaniline derivatives of the present invention are represented by the formula (1).

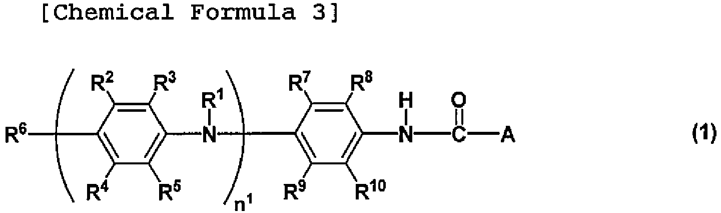

In the formula, R¹ represents a hydrogen atom or a C₁-C₂₀ alkyl group that may be substituted with Z. Z represents a halogen atom, a nitro group, a cyano group, an aldehyde group, a hydroxy group, a thiol group, a sulfonic acid group, a carboxylic acid group, a C₆-C₂₀ aryl group that may be substituted with Z', or a C₂-C₂₀ heteroaryl group that may be substituted with Z', and Z' represents a halogen atom, a nitro group, a cyano group, an aldehyde group, a hydroxy group, a thiol group, a sulfonic acid group, or a carboxylic acid group.

R² to R¹⁰ each independently represent a hydrogen atom, a halogen atom, a nitro group, a cyano group, or a C₁-C₂₀ alkyl group, C₂-C₂₀ alkenyl group, C₂-C₂₀ alkynyl group, C₆-C₂₀ aryl group, or C₂-C₂₀ heteroaryl group that may be substituted with a halogen atom.

Examples of the halogen atom include a fluorine atom, a chlorine atom, a bromine atom, or an iodine atom.

The C₁-C₂₀ alkyl group may be any of straight, branched, and cyclic C₁-C₂₀ alkyl groups, specific examples of which include: C₁-C₂₀ straight or branched alkyl groups such as methyl, ethyl, n-propyl, isopropyl, n-butyl, isobutyl, s-butyl, t-butyl, n-pentyl, n-hexyl, n-heptyl, n-octyl, n-nonyl, and n-decyl; and C₃-C₂₀ cyclic alkyl groups such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl, and bicyclodecyl.

The C₂-C₂₀ alkenyl group may be any of straight, branched, and cyclic C₂-C₂₀ alkyl groups, specific examples of which include ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pentenyl, n-1-decenyl, or n-1-eicocenyl.

The C₂-C₂₀ alkynyl group may be any of straight, branched, and cyclic C₂-C₂₀ alkynyl groups, specific examples of which include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl, or n-1-eicocynyl.

Specific examples of the C₆-C₂₀ aryl group include phenyl, 1-naphthyl, 2-naphthyl, 1-anthryl, 2-anthryl, 9-anthryl, 1-phenanthryl, 2-phenanthryl, 3-phenanthryl, 4-phenanthryl, or 9-phenanthryl.

Specific examples of the C₂-C₂₀ heteroaryl group include 2-thienyl, 3-thienyl, 2-furanyl, 3-furanyl, 2-oxazolyl, 4-oxazoyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl, or 4-pyridyl.

Among these, preferred as R¹ is a hydrogen atom or a C₁-C₁₀ alkyl group that may be substituted with Z, more preferred is a hydrogen atom or a C₁-C₄ alkyl group which may be substituted with Z, and most suitable is a hydrogen atom, taking into account the solubility of the oligoaniline derivative in an organic solvent. Note that the plurality of R¹ groups may be identical or different from one another.

In the case where R¹ is a hydrogen atom, a particularly excellent charge-transporting property can be realized when the oligoaniline derivative is used together with a dopant such as a protonic acid such as an arylsulfonic acid and a heteropoly-acid.

In addition, among these, preferred as R² to R¹⁰ is a hydrogen atom, a halogen atom, a nitro group, a cyano group, or a C₁-C₁₀ alkyl group that may be substituted with a halogen atom, and more preferred is a hydrogen atom, a halogen atom, or a C₁-C₄ alkyl group that may be substituted with a halogen atom, taking into account the solubility of the oligoaniline derivative in an organic solvent. Taking into consideration the balance between the solubility of the oligoaniline derivative in an organic solvent and the charge-transporting properties, most suitable as R² to R¹⁰ is a hydrogen atom. Note that the pluralities of R² to R⁵ may individually be identical or different from one another.

In the formula (1), A represents: a C₁-C₂₀ fluoroalkyl group, C₃-C₂₀ fluorocycloalkyl group, C₄-C₂₀ fluorobicycloalkyl group, C₂-C₂₀ fluoroalkenyl group, or C₂-C₂₀ fluoroalkynyl group that may be substituted with a cyano group, a chlorine atom, a bromine atom, an iodine atom, a nitro group, or a C₁-C₂₀ fluoroalkoxy group; a C₆-C₂₀ fluoroaryl group that may be substituted with a cyano group, a chlorine atom, a bromine atom, an iodine atom, a nitro group, a C₁-C₂₀ alkyl group, a C₁-C₂₀ fluoroalkyl group or a C₁-C₂₀ fluoroalkoxy group; a C₆-C₂₀ aryl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group; a C₇-C₂₀ fluoroaralkyl group that may be substituted with a cyano group, a chlorine atom, a bromine atom, an iodine atom, a nitro group, a C₁-C₂₀ fluoroalkoxy group, a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group; or a C₇-C₂₀ aralkyl group that may be substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group.

While the above-mentioned fluoroalkyl group is not specifically restricted so long as it is a straight or branched alkyl group wherein at least one hydrogen atom on a carbon atom is substituted with a fluorine atom, examples of the fluoroalkyl group include fluoromethyl, difluoromethyl, trifluoromethyl, 1-fluoroethyl, 2-fluoroethyl, 1,2-difluoroethyl, 1,1-difluoroethyl, 2,2-difluoroethyl, 1,1,2-trifluoroethyl, 1,2,2-trifluoroethy, 2,2,2-trifluoroethyl, 1,1,2,2-tetrafluoroethyl, 1,2,2,2-tetrafluoroethyl, 1,1,2,2,2-pentafluoroethyl, 1-fluoropropyl, 2-fluoropropyl, 3-fluoropropyl, 1,1-difluoropropyl, 1,2-difluoropropyl, 1,3-difluoropropyl, 2,2-difluoropropyl, 2,3-difluoropropyl, 3,3-difluoropropyl, 1,1,2-trifluuoropropyl, 1,1,3-trifluoropropyl, 1,2,3-trifluoropropyl, 1,3,3-trifluoropropyl, 2,2,3-trifluoropropyl, 2,3,3-trifluoropropyl, 3,3,3-trifluoropropyl, 1,1,2,2-tetrafluoropropyl, 1,1,2,3-tetrafluoropropyl, 1,2,2,3-tetrafluoropropyl, 1,3,3,3-tetrafluoropropyl, 2,2,3,3-tetrafluoropropyl, 2,3,3,3-tetrafluoropropyl, 1,1,2,2,3-pentafluoropropyl, 1,2,2,3,3-pentafluoropropyl, 1,1,3,3,3-pentafluoropropyl, 1,2,3,3,3-pentafluoropropyl, 2,2,3,3,3-pentafluoropropyl, or heptafluoropropyl.

While the above-mentioned fluorocycloalkyl group is not particularly limited so long as it is a cycloalkyl group wherein at least one hydrogen atom on a carbon atom is substituted with a fluorine atom, examples of the fluorocycloalkyl group include 1-fluorocyclopropyl, 2-fluorocyclopropyl, 2,2-difluorocyclopropyl, 2,2,3,3-tetrafluuorocyclopropyl, pentafluorocyclopropyl, 2,2-difluorocyclobutyl, 2,2,3,3-tetrafluorocyclobutyl, 2,2,3,3,4,4-hexafluorocyclobutyl, heptafluorocyclobutyl, 1-fluorocyclopentyl, 3-fluuorocyclopentyl, 3,3-difluorocyclopentyl, 3,3,4,4-tetrafluorocyclopentyl, nonafluorocyclopentyl, 1-fluorocyclohexyl, 2-fluorocyclohexyl, 4-fluorocyclohexyl, 4,4-difluorocyclohexyl, 2,2,3,3-tetrafluorocyclohexyl,
2,3,4,5,6-pentafluorocyolohexyl, or undecafluorocyclohexyl.

While the above-mentioned fluorobicycloalkyl group is not specifically restricted so long as it is a bicycloalkyl group wherein at least one hydrogen atom on a carbon atom is substituted with a fluorine atom, examples of the fluorobicycloalkyl group include
3-fluorobicyclo[1.1.0]butan-1-yl,
2,2,4,4-tetrafluorobicyclo[1.1.0]butan-1-yl,
pentafluorobicyclo[1.1.0]butan-1-yl,
3-fluorobicyclo[1.1.1]pentan-1-yl,
2,2,4,4,5-pentafluorobicyclo[1.1.1]pentan-1-yl,
2,2,4,4,5,5-hexfluorobicyclo[1.1.1]pentan-1-yl,
5-fluorobicyclo[3.1.0]hexan-6-yl,
6-fluorobicyclo[3.1.0]hexan-6-yl,
6,6-difluorobicyclo[3.1.0]hexan-2-yl,
2,2,3,3,5,5,6,6-octafluorobicyclo[2.2.0]hexan-1-yl,
1-fluorobicyclo[2.2.1]heptan-2-yl,
3-fluorobicyclo[2.2.1]heptan-2-yl,
4-fluorobicyclo[2.2.1]heptan-1-yl,
5-fluorrobicyclo[3.1.1]heptan-1-yl,
1,3,3,4,5,5,6,6,7,7-decafluorobicyclo[2.2.1]heptan-2-yl, undecafluorobicyclo[2.2.1]heptan-2-yl,
3-fluorobicyclo[2.2.2]octan-1-yl, or
4-fluorrobicyclo[2.2.2]octan-1-yl.

While the above-mentioned fluoroalkenyl group is not particularly limited so long as it is an alkenyl group wherein at least one hydrogen atom on a carbon atom is substituted with a fluorine atom, examples of the fluoroalkenyl group include 1-fluoroethenyl, 2-fluoroethenyl, 1,2-difluoroethenyl, 1,2,2-trifluoroethenyl, 2,3,3-trifluoro-1-propenyl, 3,3,3-trifluoro-1-propenyl, 2,3,3,3-tetrafluoro-1-propenyl, pentafluoro-1-propenyl, 1-fluoro-2-propenyl, 1,1-difluoro-2-propenyl, 2,3-difluoro-2-propenyl, 3,3-difluoro-2-propenyl, 2,3,3-trifluoro-2-propenyl, 1,2,3,3-tetrafluoro-2-propenyl, or pentafluoro-2-propenyl.

While the above-mentioned fluoroalkynyl group is not specifically restricted so long as it is an alkynyl group wherein at least one hydrogen atom on a carbon atom is substituted with a fluorine atom, examples of the fluoroalkynyl group include fluoroethynyl,
3-fluoro-1-propynyl, 3,3-difluoro-1-propynyl,
3,3,3-trifluoro-1-propynyl, 1-fluoro-2-propynyl, or
1,1-difluoro-2-propynyl.

While the above-mentioned fluoroaryl group is not particularly limited so long as it is an aryl group wherein at least one hydrogen atom on a carbon atom is substituted with a fluorine atom, examples of the fluoroaryl group include 2-fluorophenyl, 3-fluorophenyl, 4-fluorophenyl, 2,3-difluorophenyl, 2,4-difluorophenyl, 2,5-difluorophenyl, 2,6-difluorophenyl, 3,4-difluorophenyl, 3,5-difluorophenyl, 2,3,4-trifluorophenyl, 2,3,5-trifluorophenyl, 2,3,6-trifluorophenyl, 2,4,5-trifluorophenyl, 2,4,6-trifluorophenyl, 3,4,5-trifluorophenyl, 2,3,4,5-tetrafluorophenyl, 2,3,4,6-tetrafluorophenyl, 2,3,5,6-tetrafluorophenyl, pentafluorophenyl, 2-fluoro-1-naphthyl, 3-fluoro-1-naphthyl, 4-fluoro-1-naphthyl, 6-fluoro-1-naphthyl, 7-fluoro-1-nephthyl, 8-fluoro-1-naphthyl, 4,5-difluoro-1-naphthyl, 5,7-difluoro-1-nephthyl, 5,8-difluoro-1-naphthyl, 5,6,7,8-tetrafluoro-1-naphthyl, heptafluoro-1-naphthyl, 1-fluoro-2-naphthyl, 5-fluoro-2-naphthyl, 6-fluoro-2-naphthyl, 7-fluoro-2-naphthyl, 5,7-difluoro-2-naphthyl, or heptafluoro-2-naphthyl.

As the above-mentioned fluoroaryl group, preferred is a phenyl group that is substituted with at least three fluorine atoms and that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group, taking into consideration the balance among the solubility of the oligoaniline derivative in an organic solvent, the charge-transporting properties of the oligoaniline derivative, the availability of the materials for the oligoaniline derivative, and the like.

While the above-mentioned fluoroalkoxy group is not particularly restricted so long as it is an alkoxy group wherein at least one hydrogen atom on a carbon atom is substituted with a fluorine atom, examples of the fluoroalkoxy group include fluoromethoxy, difluoromethoxy, trifluoromethoxy, 1-fluoroethoxy, 2-fluoroethoxy, 1,2-difluoroethoxy, 1,1-difluoroethoxy, 2,2-difluoroethoxy, 1,1,2-trifluoroethoxy, 1,2,2-trifluoroethoxy, 2,2,2-trifluoroethoxy, 1,1,2,2-tetrafluoroethoxy, 1,2,2,2-tetrafluoroethoxy, 1,1,2,2,2-pentafluoroethoxy, 1-fluoropropoxy, 2-fluoropropoxy, 3-fluoropropoxy, 1,1-difluoropropoxy, 1,2-difluoropropoxy, 1,3-difluoropropoxy, 2,2-difluoropropoxy, 2,3-difluoropropoxy, 3,3-difluoropropoxy, 1,1,2-trifluoropropoxy, 1,1,3-trifluoropropoxy, 1,2,3-trifluoropropoxy, 1,3,3-trifluoropropoxy, 2,2,3-trifluoropropoxy, 2,3,3-trifluoropropoxy, 3,3,3-trifluoropropoxy, 1,1,2,2-tetrafluoropropoxy, 1,1,2,3-tetrafluoropropoxy, 1,2,2,3-tetrafluoropropoxy, 1,3,3,3-tetrafluoropropoxy, 2,2,3,3-tetrafluoropropoxy, 2,3,3,3-tetrafluoropropoxy, 1,1,2,2,3-pentafluoropropoxy, 1,2,2,3,3-pentafluoropropoxy, 1,1,3,3,3-pentafluoropropoxy, 1,2,3,3,3-pentafluoropropoxy, 2,2,3,3,3-pentafluoropropoxy, or heptafluoropropoxy.

The above-mentioned C₆-C₂₀ aryl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group (this C₆-C₂₀ aryl group will hereinafter be referred also to as a substituted aryl group, for convenience) is not particularly limited so long as it is an aryl group wherein at least one hydrogen atom on a carbon atom is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group. Examples of the substituted aryl group include
2-(trifluoromethyl)phenyl, 3-(trifluoromethyl)phenyl,
4-(trifluoromethyl)phenyl, 4-ethoxy-3-(trifluoromethyl)phenyl,
3-fluoro-4-trifluoromethylpheny1,
4-fluoro-3-trifluoromethylphenyl,
4-fluoro-2-trifluoromethylphenyl,
2-fluoro-5-(trifluoromethyl)phenyl,
3-fluoro-5-(trifluoromethyl)phenyl,
3,5-di(trifluoromethyl)phenyl,
2,4,6-tri(trifluoromethyl)phenyl, 4-(pentafluoroethyl)phenyl,
4-(3,3,3-trifluoropropyl)phenyl,
2,3,5,6-tetrafluoro-4-trifluoromethylphenyl,
4-(perfluorovinyl)phenyl, 4-(perfluoropropenyl)phenyl, or
4-(perfluorobutenyl)phenyl.

As the above-mentioned substituted aryl group, preferred is a phenyl group that is substituted with a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group (this phenyl group will hereinafter be referred to also as a substituted phenyl group, for convenience), more preferred is a phenyl group substituted with 1 to 3 trifluoromethyl groups, and further preferred is p-trifluoromethylphenyl, taking into consideration the balance among the solubility of the oligoaniline derivative in an organic solvent, the charge-transporting properties of the oligoaniline derivative, the availability of the materials for the oligoaniline derivative, and the like.

While the above-mentioned fluoroaralkyl group is not specifically restricted so long as it is an aralkyl group wherein at least one hydrogen atom on a carbon atom is substituted with a fluorine atom, examples of the fluoroaralkyl group include 2-fluorobenzyl, 3-fluorobenzyl, 4-fluorobenzyl, 2,3-difluorobenzyl, 2,4-difluorobenzyl, 2,5-difluorobenzyl, 2,6-difluorobenzyl, 3,4-difluorobenzyl, 3,5-difluorobenzyl, 2,3,4-triflurobenzyl, 2,3,5-trifluorobenzyl, 2,3,6-trifluorobenzyl, 2,4,5-trifluorobenzyl, 2,4,6-trifluorobenzyl, 2,3,4,5-tetrafluorobenzyl, 2,3,4,6-tetrafluorobenzyl, 2,3,5,6-tetrafluorobenzyl, or 2,3,4,5,6-pentafluorobenzyl.

The above-mentioned C₇-C₂₀ aralkyl group that is substituted with a C₁-C₂₀ fluoroalkyl group, a C₃-C₂₀ fluorocycloalkyl group, a C₁-C₂₀ fluorobicycloalkyl group, a C₂-C₂₀ fluoroalkenyl group, or a C₁-C₂₀ fluoroalkynyl group and that may be substituted with a cyano group, a halogen atom, or a C₁-C₂₀ fluoroalkoxy group is not particularly limited so long as it is an aralkyl group wherein at least one hydrogen atom on a carbon atom is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group. Examples of this aralkyl group include 2-trifluoromethylbenzyl, 3-trifluoromethylbenzyl, 4-trifluoromethylbenzyl, 2,4-di(trifluoromethyl)benzyl, 2,5-di(trifluoromethyl)benzyl, 2,6-di(trifluoromethyl)benzyl, 3,5-di(trifluoromethyl)benzyl, or
2,4,6-tri(trifluoromethyl)benzyl.

Among these, preferred as A is the above-mentioned C₁-C₂₀ fluoroalkyl group that may be substituted, the above-mentioned C₆-C₂₀ fluoroaryl group that may be substituted or the above-mentioned substituted aryl group, more preferred as A is the above-mentioned C₆-C₂₀ fluoroaryl group that may be substituted or the above-mentioned aryl group that may be substituted, still more preferred as A is the above-mentioned fluorophenyl group that may be substituted or the above-mentioned substituted phenyl group, and further preferred as A is the above-mentioned trifluorophenyl group that may be substituted, the above-mentioned tetrafluorophenyl group that may be substituted, the above-mentioned pentafluorophenyl group that may be substituted, or a phenyl group that is substituted with 1 to 3 trifluoromethyl groups.

Specific examples of a preferable group for use as A will be given below, but A is not limited to these exemplary groups.

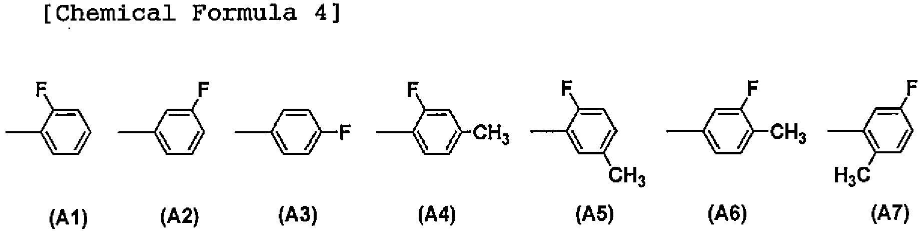

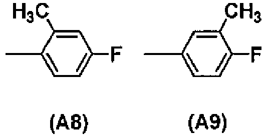

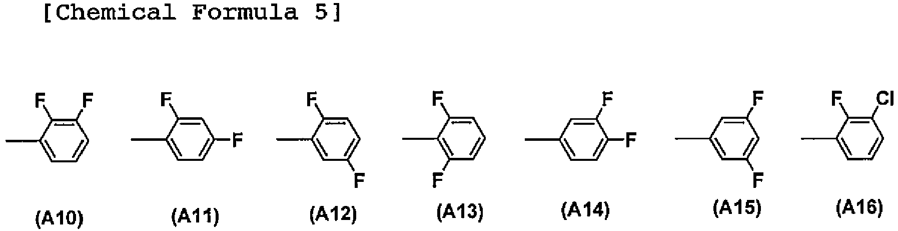

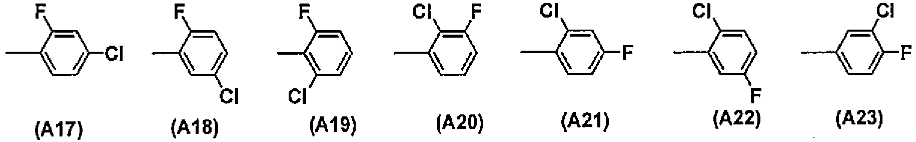

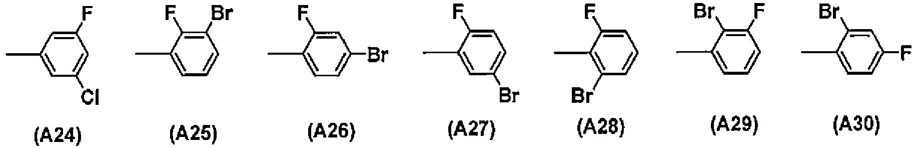

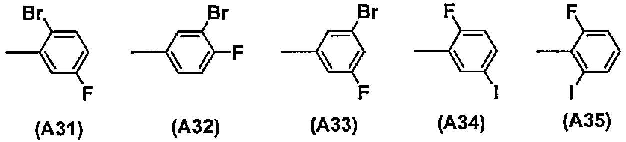

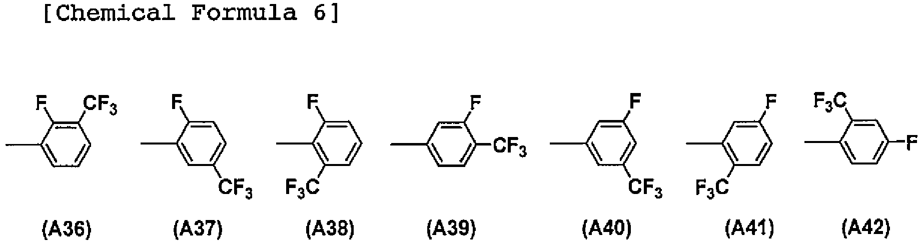

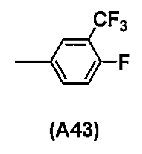

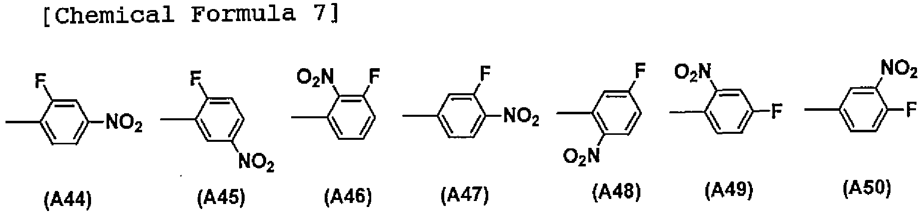

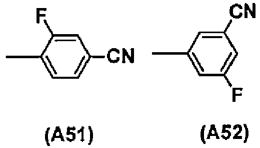

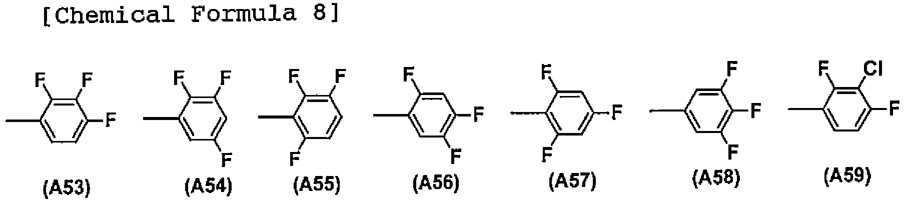

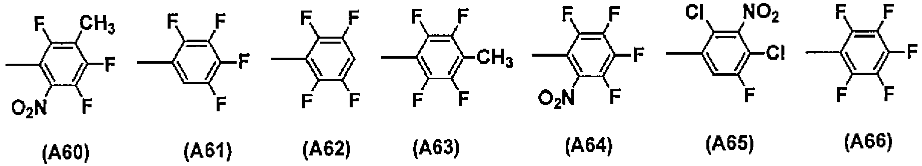

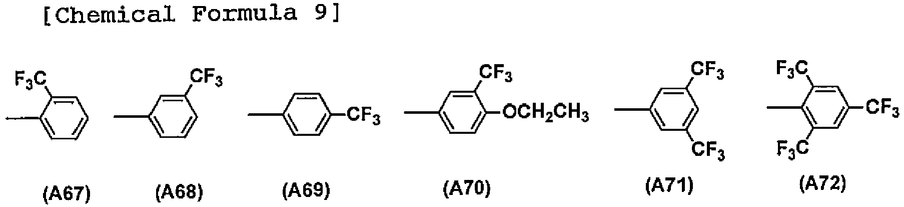

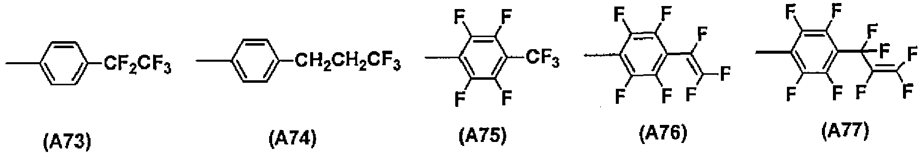

Besides, in the formula (1), while letter n¹ is an integer of 1 to 20, n¹ is preferably up to 10, more preferably up to 8, still more preferably up to 5, and further preferably up to 4, from the viewpoint of enhancing the solubility of the oligoaniline derivative in a solvent. In addition, from the viewpoint of enhancing the charge-transporting properties of the oligoaniline derivative, n¹ is preferably at least 2, more preferably at least 3, and, taking into account the balance between the solubility and the charge-transporting properties, n¹ is most suitably 3.

### [Synthesizing Method for Oligoaniline Derivatives]

The oligoaniline derivatives of the present invention can be synthesized by reacting an amine compound represented by the formula (2) with a fluorine-containing acid halide represented by the formula (3). 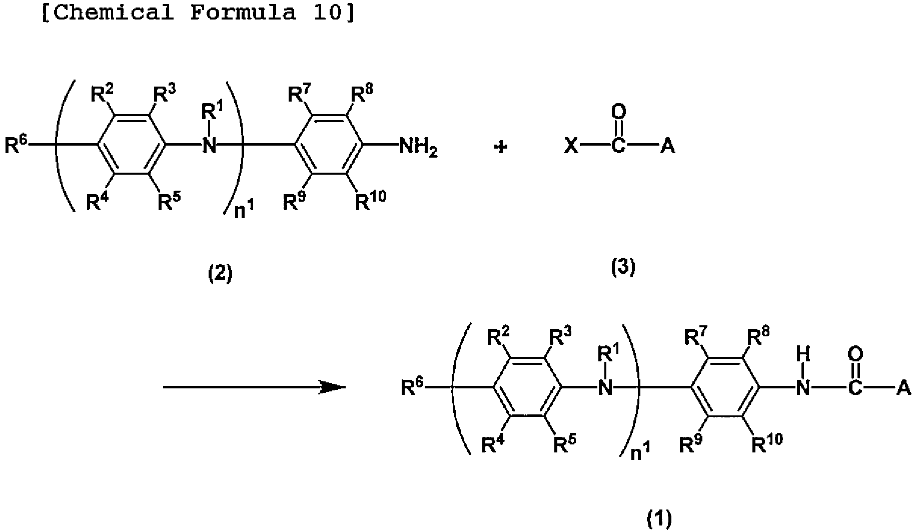 (In the formula, R¹ to R¹⁰, A, and letter n¹ are the same as defined above. X represents a halogen atom such as a fluorine atom, a chlorine atom, a bromine atom, and an iodine atom, and is preferably a chlorine atom or a bromine atom.)

Examples of the amine compound represented by the formula (2) include, but are not limited to, those represented by the following formula.

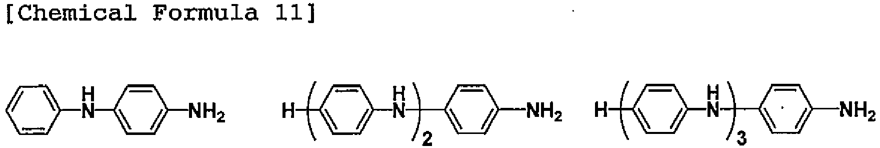

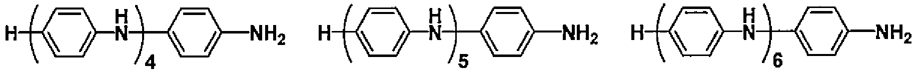

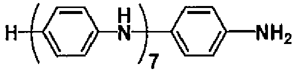

Examples of the fluorine-containing acid halide represented by the formula (3) include, but are not limited to,
2-fluorobenzoyl chloride, 3-fluorobenzoyl chloride,
4-fluorobenzoyl chloride, 2-fluoro-4-methylbenzoyl chloride, 2-fluoro-5-methylbenzoyl chloride,
3-fluoro-4-methylbenzoyl chloride,
3-fluoro-6-methylbenzoyl chloride,
4-fluoro-2-methylbenzoyl chloride,
4-fluoro-3-methylbenzoyl chloride,
2,3-difluorobenzoyl chloride, 2,4-difluorobenzoyl chloride, 2,5-difluorobenzoyl chloride, 2,6-difluorobenzoyl chloride, 3,4-difluorobenzoyl chloride, 3,5-difluorobenzoyl chloride, 3-chloro-2-fluorobenzoyl chloride,
4-chloro-2-fluorobenzoyl chloride,
5-chloro-2-fluorobenzoyl chloride,
2-chloro-6-fluorobenzoyl chloride,
2-chloro-3-fluorobenzoyl chloride,
2-chloro-4-fluorobenzoyl chloride,
2-chloro-5-fluorobenzoyl chloride,
3-chloro-4-fluorobenzoyl chloride,
3-chloro-5-fluorobenzoyl chloride,
3-bromo-2-fluorobenzoyl chloride,
4-bromo-2-fluorobenzoyl chloride,
5-bromo-2-fluorobenzoyl chloride,
2-bromo-6-fluorobenzoyl chloride,
2-bromo-3-fluorobenzoyl chloride,
2-bromo-4-fluorobenzoyl chloride,
2-bromo-5-fluorobenzoyl chloride,
3-bromo-4-fluorobenzoyl chloride,
3-bromo-5-fluorobenzoyl chloride,
2-fluoro-5-iodobenzoyl chloride,
2-fluuoro-6-iodobenzoyl chloride,
2-fluoro-3-(trifluoromethyl)benzoyl chloride,
2-fluoro-5-(trifluoromethyl)benzoyl chloride,
2-fluoro-6-(trifluoromethyl)benzoyl chloride,
3-fluoro-4-(trifluoromethyl)benzoyl chloride,
3-fluoro-5-(trifluoromethyl)benzoyl chloride,
3-fluoro-6-(trifluoromethyl)benzoyl chloride,
4-fluoro-2-(trifluoromethyl)benzoyl chloride,
4-fluoro-3-(trifluoromethyl)benzoyl chloride,
2-fluoro-4-nitrobenzoyl chloride,
2-fluoro-5-nitrobenzoyl chloride,
3-fluoro-2-nitrobenzoyl chloride,
3-fluoro-4-nitrobenzoyl chloride,
3-fluoro-6-nitrobenzoyl chloride,
4-fluoro-2-nitrobenzoyl chloride,
4-fluoro-3-nitrobenzoyl chloride,
4-cyano-2-fluorobenzoyl chloride,
3-cyano-5-fluorobenzoyl chloride,
2,3,4-trifluorobenzoyl chloride,
2,3,5-trifluorobenzoyl chloride,
2,3,6-trifluorobenzoyl chloride,
2,4,5-trifluorobenzoyl chloride,
2,4,6-trifluorobenzoyl chloride,
3,4,5-trifluorobenzoyl chloride,
4-chloro-2,4-difluorobenzoyl chloride,
2,4-dichloro-5-fluoro-4-nitrobenzoyl chloride,
2,4,5-trifluoro-3-methyl-6-nitrobenzoyl chloride,
2,3,4,5-tetrafluorobenzoyl chloride,
2,3,5,6-tetrafluorobenzoyl chloride,
2,3,5,6-tetrafluoro-4-methyl-benzoyl chloride,
2,3,4,5-tetrafluoro-6-nitrobenzoyl chloride,
2,3,4,5,6-pentafluorobenzoyl chloride,
2-(trifluoromethyl)benzoyl chloride,
3-(trifluoromethyl)benzoyl chloride,
4-(trifluoromethyl)benzoyl chloride,
3-trifluoromethyl-4-ethoxybenzoyl chloride,
3,5-bis(trifluoromethyl)benzoyl chloride,
2,4,6-tris(trifluoromethyl)benzoyl chloride,
4-(pentafluoroethyl)benzoyl chloride,
4-(3-tetrafluoropropyl)benzoyl chloride,
2,3,5,6-tetrafluoro-4-(trifluoromethyl)benzoyl chloride,
2,3,5,6-tetrafluoro-4-(trifluorovinyl)benzoyl chloride, or
2,3,5,6-tetrafluoro-4-(pentafluoroallyl)benzoyl chloride.

A reaction solvent is preferably an aprotic polar organic solvent, examples of which include N,N-dimethylformamide, N,N-dimethylacetamide, N-methylpyrrolidone, 1,3-dimethyl-2-imidazolidinone, dimethyl sulfoxide, tetrahydrofuran, or dioxane. From the viewpoint of ease of removal of the reaction solvent after the reaction, the reaction solvent is preferably N,N-dimethylformamide, N,N-dimethylacetamide, tetrahydrofuran, or dioxane.

A reaction temperature can normally be from -50°C up to the boiling point of the solvent used, and is preferably in the range of 0°C to 140°C. A reaction time is normally 0.1 hour to 100 hours.

After the reaction is completed, an after-treatment is conducted according to the usual method, whereby the desired oligoaniline derivative can be obtained.

Note that the fluorine-containing acid halide represented by the formula (3) can be obtained by reacting the corresponding fluorine-containing carboxylic acid with an electrophilic halogenating agent such as, for example, thionyl chloride, oxalyl chloride, phosphoryl chloride, sulfuryl chloride, phosphorus trichloride, or phosphorus pentachloride.

In addition, the corresponding fluorine-containing carboxylic acid may be a commercially available one, or can be synthesized by a known method (for example, the methods described in JP-A H09-67303, JP-A H09-67304, or JP-A 2002-284733).

Specific examples of the oligoaniline derivatives represented by the formula (1) will now be set forth below, but they are not restrictive. Note that "R¹ to R¹⁰," "A," and letter "n¹" in the table represent designations in the formula (1) which are relevant to each compound set forth in each line; for example, each of the compound represented by formula (E1) and the compound represented by formula (E138) is as follows.

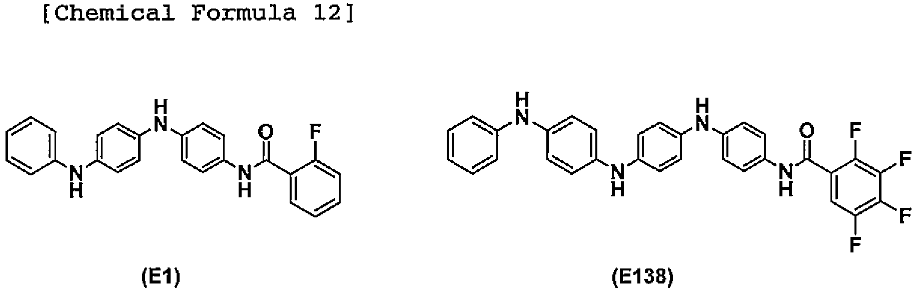

**[Table 1]**

| Compound | R¹ to R¹⁰ | A | n¹ | | Compound | R¹ to R¹⁰ | A | n¹ | | Compound | R¹ to R¹⁰ | A | n¹ |
|---|---|---|---|---|---|---|---|---|---|---|---|---|---|
| (E1) | H | (A1) | 2 | | (E29) | H | (A29) | 2 | | (E57) | H | (A57) | 2 |
| (E2) | H | (A2) | 2 | | (E30) | H | (A30) | 2 | | (E58) | H | (A58) | 2 |
| (E3) | H | (A3) | 2 | | (E31) | H | (A31) | 2 | | (E59) | H | (A59) | 2 |
| (E4) | H | (A4) | 2 | | (E32) | H | (A32) | 2 | | (E60) | H | (A60) | 2 |
| (E5) | H | (A5) | 2 | | (E33) | H | (A33) | 2 | | (E61) | H | (A61) | 2 |
| (E6) | H | (A6) | 2 | | (E34) | H | (A34) | 2 | | (E62) | H | (A62) | 2 |
| (E7) | H | (A7) | 2 | | (E35) | H | (A35) | 2 | | (E63) | H | (A63) | 2 |
| (E8) | H | (A8) | 2 | | (E36) | H | (A36) | 2 | | (E64) | H | (A64) | 2 |
| (E9) | H | (A9) | 2 | | (E37) | H | (A37) | 2 | | (E65) | H | (A65) | 2 |
| (E10) | H | (A10) | 2 | | (E38) | H | (A38) | 2 | | (E66) | H | (A66) | 2 |
| (E11) | H | (A11) | 2 | | (E39) | H | (A39) | 2 | | (E67) | H | (A67) | 2 |
| (E12) | H | (A12) | 2 | | (E40) | H | (A40) | 2 | | (E68) | H | (A68) | 2 |
| (E13) | H | (A13) | 2 | | (E41) | H | (A41) | 2 | | (E69) | H | (A69) | 2 |
| (E14) | H | (A14) | 2 | | (E42) | H | (A42) | 2 | | (E70) | H | (A70) | 2 |
| (E15) | H | (A15) | 2 | | (E43) | H | (A43) | 2 | | (E71) | H | (A71) | 2 |
| (E16) | H | (A16) | 2 | | (E44) | H | (A44) | 2 | | (E72) | H | (A72) | 2 |
| (E17) | H | (A17) | 2 | | (E45) | H | (A45) | 2 | | (E73) | H | (A73) | 2 |
| (E18) | H | (A18) | 2 | | (E46) | H | (A46) | 2 | | (E74) | H | (A74) | 2 |
| (E19) | H | (A19) | 2 | | (E47) | H | (A47) | 2 | | (E75) | H | (A75) | 2 |
| (E20) | H | (A20) | 2 | | (E48) | H | (A48) | 2 | | (E76) | H | (A76) | 2 |
| (E21) | H | (A21) | 2 | | (E49) | H | (A49) | 2 | | (E77) | H | (A77) | 2 |
| (E22) | H | (A22) | 2 | | (E50) | H | (A50) | 2 | | | | | |
| (E23) | H | (A23) | 2 | | (E51) | H | (A51) | 2 | | | | | |
| (E24) | H | (A24) | 2 | | (E52) | H | (A52) | 2 | | | | | |
| (E25) | H | (A25) | 2 | | (E53) | H | (A53) | 2 | | | | | |
| (E26) | H | (A26) | 2 | | (E54) | H | (A54) | 2 | | | | | |
| (E27) | H | (A27) | 2 | | (E55) | H | (A55) | 2 | | | | | |
| (E28) | H | (A28) | 2 | | (E56) | H | (A56) | 2 | | | | | |

**[Table 2]**

| Compound | R¹ to R¹⁰ | A | n¹ | | Compound | R¹ to R¹⁰ | A | n¹ | | Compound | R¹ to R¹⁰ | A | n¹ |
|---|---|---|---|---|---|---|---|---|---|---|---|---|---|
| (E78) | H | (A1) | 3 | | (E106) | H | (A29) | 3 | | (E134) | H | (A57) | 3 |
| (E79) | H | (A2) | 3 | | (E107) | H | (A30) | 3 | | (E135) | H | (A58) | 3 |
| (E80) | H | (A3) | 3 | | (E108) | H | (A31) | 3 | | (E136) | H | (A59) | 3 |
| (E81) | H | (A4) | 3 | | (E109) | H | (A32) | 3 | | (E137) | H | (A60) | 3 |
| (E82) | H | (A5) | 3 | | (E110) | H | (A33) | 3 | | (E138) | H | (A61) | 3 |
| (E83) | H | (A6) | 3 | | (E111) | H | (A34) | 3 | | (E139) | H | (A62) | 3 |
| (E84) | H | (A7) | 3 | | (E112) | H | (A35) | 3 | | (E140) | H | (A63) | 3 |
| (E85) | H | (A8) | 3 | | (E113) | H | (A36) | 3 | | (E141) | H | (A64) | 3 |
| (E86) | H | (A9) | 3 | | (E114) | H | (A37) | 3 | | (E142) | H | (A65) | 3 |
| (E87) | H | (A10) | 3 | | (E115) | H | (A38) | 3 | | (E143) | H | (A66) | 3 |
| (E88) | H | (A11) | 3 | | (E116) | H | (A39) | 3 | | (E144) | H | (A67) | 3 |
| (E89) | H | (A12) | 3 | | (E117) | H | (A40) | 3 | | (E145) | H | (A68) | 3 |
| (E90) | H | (A13) | 3 | | (E118) | H | (A41) | 3 | | (E146) | H | (A69) | 3 |
| (E91) | H | (A14) | 3 | | (E119) | H | (A42) | 3 | | (E147) | H | (A70) | 3 |
| (E92) | H | (A15) | 3 | | (E120) | H | (A43) | 3 | | (E148) | H | (A71) | 3 |
| (E93) | H | (A16) | 3 | | (E121) | H | (A44) | 3 | | (E149) | H | (A72) | 3 |
| (E94) | H | (A17) | 3 | | (E122) | H | (A45) | 3 | | (E150) | H | (A73) | 3 |
| (E95) | H | (A18) | 3 | | (E123) | H | (A46) | 3 | | (E151) | H | (A74) | 3 |
| (E96) | H | (A19) | 3 | | (E124) | H | (A47) | 3 | | (E152) | H | (A75) | 3 |
| (E97) | H | (A20) | 3 | | (E125) | H | (A48) | 3 | | (E153) | H | (A76) | 3 |
| (E98) | H | (A21) | 3 | | (E126) | H | (A49) | 3 | | (E154) | H | (A77) | 3 |
| (E99) | H | (A22) | 3 | | (E127) | H | (A50) | 3 | | | | | |
| (E100) | H | (A23) | 3 | | (E128) | H | (A51) | 3 | | | | | |
| (E101) | H | (A24) | 3 | | (E129) | H | (A52) | 3 | | | | | |
| (E102) | H | (A25) | 3 | | (E130) | H | (A53) | 3 | | | | | |
| (E103) | H | (A26) | 3 | | (E131) | H | (A54) | 3 | | | | | |
| (E104) | H | (A27) | 3 | | (E132) | H | (A55) | 3 | | | | | |
| (E105) | H | (A28) | 3 | | (E133) | H | (A56) | 3 | | | | | |

**[Table 3]**

| Compound | R¹ to R¹⁰ | A | n¹ | | Compound | R¹ to R¹⁰ | A | n¹ | | Compound | R¹ to R¹⁰ | A | n¹ |
|---|---|---|---|---|---|---|---|---|---|---|---|---|---|
| (E155) | H | (A1) | 4 | | (E183) | H | (A29) | 4 | | (E211) | H | (A57) | 4 |
| (E156) | H | (A2) | 4 | | (E184) | H | (A30) | 4 | | (E212) | H | (A58) | 4 |
| (E157) | H | (A3) | 4 | | (E185) | H | (A31) | 4 | | (E213) | H | (A59) | 4 |
| (E158) | H | (A4) | 4 | | (E186) | H | (A32) | 4 | | (E214) | H | (A60) | 4 |
| (E159) | H | (A5) | 4 | | (E187) | H | (A33) | 4 | | (E215) | H | (A61) | 4 |
| (E160) | H | (A6) | 4 | | (E188) | H | (A34) | 4 | | (E216) | H | (A62) | 4 |
| (E161) | H | (A7) | 4 | | (E189) | H | (A35) | 4 | | (E217) | H | (A63) | 4 |
| (E162) | H | (A8) | 4 | | (E190) | H | (A36) | 4 | | (E218) | H | (A64) | 4 |
| (E163) | H | (A9) | 4 | | **(E191)** | H | (A37) | 4 | | (E219) | H | (A65) | 4 |
| (E164) | H | (A10) | 4 | | **(E192)** | H | (A38) | 4 | | (E220) | H | (A66) | 4 |
| (E165) | H | (A11) | 4 | | **(E193)** | H | (A39) | 4 | | (E221) | H | (A67) | 4 |
| (E166) | H | (A12) | 4 | | **(E194)** | H | **(A40)** | 4 | | (E222) | H | (A68) | 4 |
| (E167) | H | (A13) | 4 | | **(E195)** | H | (A41) | 4 | | (E223) | H | (A69) | 4 |
| (E168) | H | (A14) | 4 | | **(E196)** | H | (A42) | 4 | | (E224) | H | (A70) | 4 |
| (E169) | H | (A15) | 4 | | (E197) | H | (A43) | 4 | | (E225) | H | (A71) | 4 |
| (E170) | H | (A16) | 4 | | (E198) | H | (A44) | 4 | | (E226) | H | (A72) | 4 |
| (E171) | H | (A17) | 4 | | **(E199)** | H | (A45) | 4 | | (E227) | H | (A73) | 4 |
| (E172) | H | (A18) | 4 | | **(E200)** | H | (A46) | 4 | | (E228) | H | (A74) | 4 |
| (E173) | H | (A19) | 4 | | **(E201)** | H | (A47) | 4 | | (E229) | H | (A75) | 4 |
| (E174) | H | (A20) | 4 | | **(E202)** | H | (A48) | 4 | | (E230) | H | (A76) | 4 |
| (E175) | H | (A21) | 4 | | (E203) | H | (A49) | 4 | | (E231) | H | (A77) | 4 |
| (E176) | H | (A22) | 4 | | (E204) | H | (A50) | 4 | | | | | |
| (E177) | H | (A23) | 4 | | **(E205)** | H | (A51) | 4 | | | | | |
| (E178) | H | (A24) | 4 | | **(E206)** | H | (A52) | 4 | | | | | |
| (E179) | H | **(A25)** | 4 | | (E207) | H | (A53) | 4 | | | | | |
| (E180) | H | (A26) | 4 | | **(E208)** | H | (A54) | 4 | | | | | |
| (E181) | H | (A27) | 4 | | (E209) | H | (A55) | 4 | | | | | |
| (E182) | H | (A28) | 4 | | **(E210)** | H | (A56) | 4 | | | | | |

### [Charge-transporting Varnishes]

The charge-transporting varnishes of the present invention each contain the oligoaniline derivative and an organic solvent.

### [Organic Solvent]

As the organic solvent to be used in preparing the charge-transporting varnish, there can be used a high-solvency solvent capable of favorably dissolving the charge-transporting substance and the dopant therein.

Examples of such a high-solvency solvent include, but are not limited to, such organic acids as cyclohexane, N,N-dimethylformamide, N,N-dimethylacetamide, N,N-dimethylisobutylamide, N-methylpyrrolidone, and 1,3-dimethyl-2-imidazolidinone. These solvents can be used either singly or in mixture of at least two of them. The amount of the high-solvency solvent to be used may be 5% to 100% by weight, based on the total amount of the solvents used in the varnish.

Note that it is preferable that both the charge-transporting substance and the dopant are completely dissolved or uniformly dispersed, and it is more preferable that both of them are completely dissolved.

Besides, in the present invention, the varnish can contain at least one high-viscosity organic solvent having a viscosity of 10 mPa·s to 200 mPa·s, particularly 35 mPa·s to 150 mPa·s at 25°C and a boiling point of 50°C to 300°C, particularly 150°C to 250°C at normal pressure (atmospheric pressure). The addition of such a solvent makes it easy to control the viscosity of the varnish and makes it possible to prepare a varnish which accords to the coating method used and which gives a highly flat thin film with good reproducibility.

Examples of the high-viscosity organic solvent include, but are not limited to, cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol, or hexylene glycol.

The proportion of the high-viscosity organic solvent added, based on the total amount of the solvents used in the varnish of the present invention, is preferably in such a range that solid precipitation does not occur. The proportion of the high-viscosity organic solvent added is preferably 5% to 90% by weight, provided that solid precipitation does not occur.

Further, for the purpose of enhancing wettability with respect to a substrate, control of surface tension of the solvent, control of polarity, or control of boiling point, other solvents can also be mixed in a proportion of 1% to 90% by weight, preferably 1% to 50% by weight, based on the total amount of the solvents used in the varnish.

Examples of such solvents include, but are not limited to, propylene glycol monomethyl ether, ethylene glycol monobutyl ether, diethylene glycol diethyl ether, diethylene glycol monomethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate, or n-hexyl acetate. These solvents can be used either singly or in mixture of at least two of them.

The viscosity of the varnish of the present invention is appropriately set according to the thickness or the like of the thin film to be produced and the concentration of solid components. Normally, the viscosity is 1 mPa·s to 50 mPa·s at 25°C. In addition, the concentration of solid components of the charge-transporting varnish in the present invention is appropriately set taking into account the viscosity and surface tension of the varnish, the thickness of the thin film to be produced, and the like. Normally, the concentration of solid components is approximately 0.1% to 10.0% by weight, and, from the viewpoint of enhancing the coating properties of the varnish, the concentration is preferably 0.5% to 5.0% by weight, more preferably 1.0% to 3.0% by weight. Note that the solid components are the components of the varnish excluding the organic solvents.

### [Dopant]

The charge-transporting varnishes of the present invention may each contain a dopant according to the use of the thin film to be obtained, for the purpose of, for example, enhancing the charge-transporting capability thereof. The dopant is not particularly limited so long as it is dissolved in at least one solvent used in the varnish. As the dopant, either of an inorganic dopant and an organic dopant can be used.

Where the dopant is used, the amount of the dopant compounded, in material amount ratio (molar ratio) based on the oligoaniline derivative, is preferably from 0.0001 to 1, more preferably from 0.001 to 0.5, and further preferably from 0.01 to 0.2.

Examples of the inorganic dopant include: inorganic acids such as hydrogen chloride, sulfuric acid, nitric acid, and phosphoric acid; metal halides such as aluminum(III) chloride (AlCl₃), titanium(IV) tetrachloride (TiCl₄), boron tribromide (BBr₃), boron trifluoride ether complex (BF₃·OEt₂), iron(III) chloride (FeCl₃), copper(II) chloride (CuCl₂). antimony(V) pentachloride (SbCl₅), antimony(V) pentafluoride (SbF₅), arsenic(V) pentafluoride (AsF₅), phosphorus pentafluoride (PF₅), and tris(4-bromophenyl)aluminum hexachloroantimonate (TBPAH); halogens such as Cl₂, Br₂, I₂, ICl, ICl₃, IBr, and IF₄; and heteropoly-acids such as phosphomolybdic acid and phosphotungstic acid.

Examples of the organic dopant include arylsulfone compounds such as benzenesulfonic acid, tosic acid, p-styrenesulfonic acid, 2-naphthalenesulfonic acid,
4-hydroxybenzenesulfonic acid, 5-sulfosalicylic acid,
p-dodecylbenzenesulfonic acid, dihexylbenzenesulfonic acid,
2,5-dihexylbenzenesulfonic acid,
dibutylnaphthalenesulfonic acid,
6,7-dibutyl-2-naphthalenesulfonic acid,
dodecylnaphthalenesulfonic acid,
3-dodecyl-2-naphthalenesulfonic acid,
hexylnaphthalenesulfonic acid,
4-hexyl-1-naphthalenesulfonic acid,
octylnaphthalenesulfonic acid,
2-octyl-1-naphthalenesulfonic acid,
hexylnaphthalenesulfonic acid,
7-hexyl-1-naphthalenesulfonic acid,
6-hexyl-2-naphthalenesulfonic acid,
dinonylnaphthalenesulfonic acid,
2,7-dinonyl-4-naphthalenesulfonic acid,
dinonylnaphthalenedisulfonic acid,
2,7-dinonyl-4,5-naphthalenedisulfonic acid,
the 1,4-benzodioxanedisulfonic acid compounds described in WO 2005/000832,
the arylsulfonic acid compounds described in WO 2006/025342,
the arylsulfonic acid compounds described in WO 2009/096352, and polystyrenesulfonic acid. These inorganic and organic dopants may be used either singly or in combination of at least two of them.

Besides, arylsulfonic acid compounds represented by formula (4) can also be preferably used as the dopant. 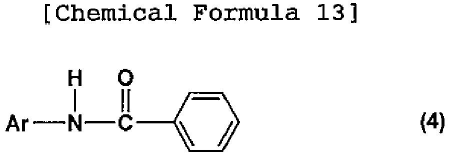 [In the formula, Ar is a group represented by formula (5) or formula (6). 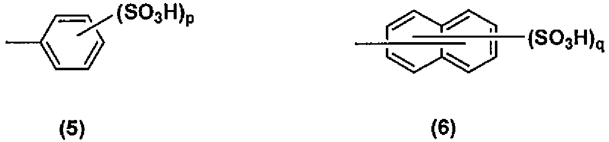 (In the formula, letter p represents an integer of 1 to 5, and letter q represents an integer of 1 to 7.)]

The arylsulfonic acid compounds represented by the formula (4) can be obtained by reacting an amine compound represented by formula (5) with an acid halide represented by formula (6) to obtain an arylsulfonic acid salt represented by formula (4'), and subjecting the salt to an ion exchange treatment. 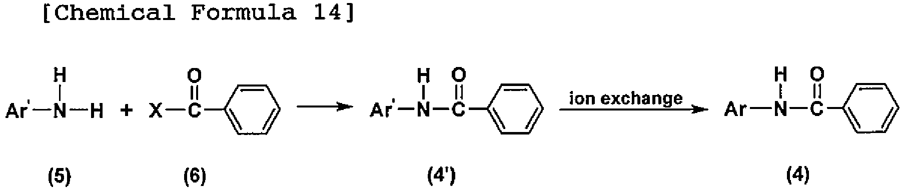 [In the formulas, Ar and X are the same as defined above. Ar' represents a group represented by formula (5') or formula (6'). 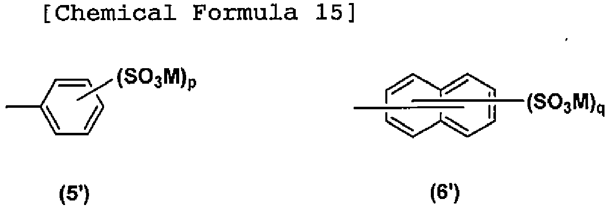 (In the formulas, letter p and letter q are the same as defined above. M represents an alkali metal atom such as sodium and potassium.)]

Examples of the amine compound represented by the formula (5) include, but are not limited to,
disodium aniline-2,4-disulfonate,
disodium aniline-2,5-disulfonate,
disodium 8-amino-naphthalene-1,5-disulfonate,
disodium 2-amino-naphthalene-1,5-disulfonate,
disodium 2-amino-naphthalene-3,6-disulfonate,
disodium 7-aminonaphthalene-1,5-disulfonate,
disodium 7-aminonaphthalene-2,4-disulfonate, or
disodium 7-aminonaphthalene-1,3-disulfonate. Note that the amine compound represented by the formula (5) to be used may be a hydrate.

Examples of the acid halide represented by the formula (6) include benzoyl chloride or benzoyl bromide.

A reaction solvent is preferably an aprotic polar organic solvent, examples of which include
N,N-dimethylformamide, N,N-dimethylacetamide,
N-methylpyrrolidone, 1,3-dimethyl-2-imidazolidinone,
dimethyl sulfoxide, tetrahydrofuran, or dioxane. From the viewpoint of ease of removal of the reaction solvent after the reaction, the reaction solvent is preferably N,N-dimethylformamide, N,N-dimethylacetamide, tetrahydrofuran, or dioxane.

A reaction temperature can normally be from -50°C up to the boiling point of the solvent used, and is preferably in the range from 0°C to 140° C. A reaction time is normally 0.1 hour to 100 hours.

After the reaction is completed, the arylsulfonic acid salt represented by the formula (4') is collected by filtration and distilling-off of the reaction solvent, after which the sulfonate is protonated, for example, by use of a cation exchange resin, whereby the arylsulfonic acid compound represented by the formula (1) can be produced.

Note that the acid halide represented by the formula (6) can be obtained by reacting the corresponding carboxylic acid with an electrophilic halogenating agent such as, for example, thionyl chloride, oxalyl chloride, phosphoryl chloride, sulfuryl chloride, phosphorus trichloride, or phosphorus pentachloride.

As the dopant, arylsulfonic acid compounds and heteropoly-acids are particularly preferred, since they show good solubility in organic solvents when used together with the oligoaniline derivative of the present invention.

Specific examples of a preferable dopant are given below, but they are not restrictive.

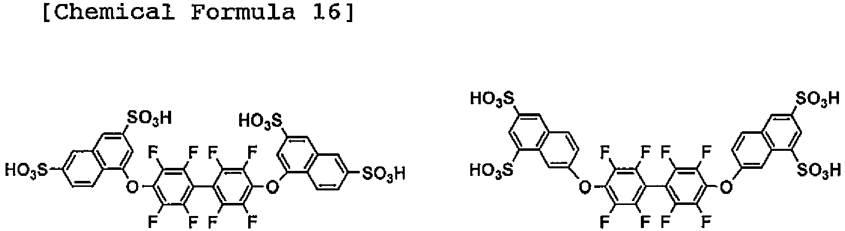

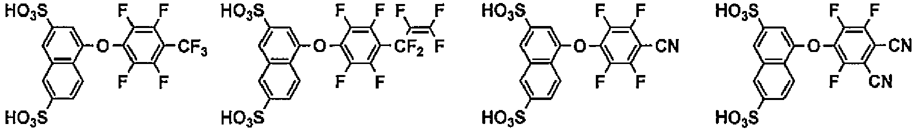

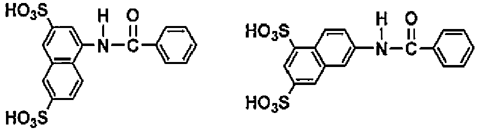

### [Other Components]

The charge-transporting varnishes of the present invention may contain an organosilane compound. Examples of the organosilane compound include dialkoxysilane compounds, trialkoxysilane compounds, and tetraalkoxysilane compounds. These may be used either singly or in combination of at least two of them. With the organosilane compound contained in the charge-transporting varnish, enhancement of hole injection capability of the charge-transporting thin film obtained and the like can be expected when the thin film is used as a hole injection layer of an organic EL element.

Among others, the organosilane compound is preferably a dialkoxysilane compound or a trialkoxysilane compound, and more preferably a trialkoxysilane compound.

Examples of these alkoxysilane compounds include those represented by formulas (7) to (9).

SiR'₂(OR)₂ (7)

SiR'(OR)₃ (8)

Si(OR)₄ (9)

In the formulas, R groups each independently represent a C₁-C₂₀ alkyl group that may be substituted with Z¹⁰¹, a C₂-C₂₀ alkenyl group that may be substituted with Z¹⁰¹, a C₂-C₂₀ alkynyl group that may be substituted with Z¹⁰¹, a C₆-C₂₀ aryl group that may be substituted with Z¹⁰², or a C₂-C₂₀ heteroaryl group that may be substituted with Z¹⁰².

R' groups each independently represent a C₁-C₂₀ alkyl group that may be substituted with Z¹⁰³, a C₂-C₂₀ alkenyl group that may be substituted with Z¹⁰³, a C₂-C₂₀ alkynyl group that may be substituted with Z¹⁰³, a C₆-C₂₀ aryl group that may be substituted with **Z**¹⁰⁴, or a C₂-C₂₀ heteroaryl group that may be substituted with Z¹⁰⁴.

Z¹⁰¹ represents a halogen atom, a C₆-C₂₀ aryl group that may be substituted with Z¹⁰⁵, or a C₂-C₂₀ heteroaryl group that may be substituted with Z¹⁰⁵.

Z¹⁰² represents a halogen atom, a C₁-C₂₀ alkyl group that may be substituted with Z¹⁰⁵, a C₂-C₂₀ alkenyl group that may be substituted with Z¹⁰⁵, or a C₂-C₂₀ alkynyl group that may be substituted with Z¹⁰⁵.

Z¹⁰³ represents a halogen atom, a C₆-C₂₀ aryl group that may be substituted with Z¹⁰⁵, a C₂-C₂₀ heteroaryl group that may be substituted with Z¹⁰⁵, an epoxycyclohexyl group, a glycidoxy group, a methacryloxy group, an acryloxy group, a ureido group (-NHCONH₂), a thiol group, an isocyanate group (-NCO), an amino group, a -NHY¹⁰¹ group, or a -NY¹⁰²Y¹⁰³ group.

Z¹⁰⁴ represents a halogen atom, a C₁-C₂₀ alkyl group that may be substituted with Z¹⁰⁵, a C₂-C₂₀ alkenyl group that may be substituted with Z¹⁰⁵, a C₂-C₂₀ alkynyl group that may be substituted with Z¹⁰⁵, an epoxycyclohexyl group, a glycidoxy group, a methacryloxy group, an acryloxy group, a ureido group (-NHCONH₂), a thiol group, an isocyanate group (-NCO), an amino group, a -NHY¹⁰¹ group, or a -NY¹⁰²Y¹⁰³ group.

Y¹⁰¹ to Y¹⁰³ each independently represent a C₁-C₂₀ alkyl group that may be substituted with Z¹⁰⁵, a C₂-C₂₀ alkenyl group that may be substituted with Z¹⁰⁵, a C₂-C₂₀ alkynyl group that may be substituted with Z¹⁰⁵, a C₆-C₂₀ aryl group that may be substituted with Z¹⁰⁵ or a C₂-C₂₀ heteroaryl group that may be substituted with Z¹⁰⁵.

Z¹⁰⁵ represents a halogen atom, an amino group, a nitro group, a cyano group, or a thiol group.

Examples of the halogen atom, the C₁-C₂₀ alkyl group, the C₂-C₂₀ alkenyl group, the C₂-C₂₀ alkynyl group, the C₆-C₂₀ aryl group, and the C₂-C₂₀ heteroaryl group in the formulas (7) to (9) include the same groups as above-mentioned.

In R and R', the numbers of carbon atoms in the alkyl group, the alkenyl group, and the alkynyl group are preferably up to 10, more preferably up to 6, and still more preferably up to 4. Besides, the numbers of carbon atoms in the aryl group and the heteroaryl group are preferably up to 14, more preferably up to 10, and still more preferably up to 6.

As R, preferred is the C₁-C₂₀ alkyl group that may be substituted with Z¹⁰¹, the C₂-C₂₀ alkenyl group that may be substituted with Z¹⁰¹, or the C₆-C₂₀ aryl group that may be substituted with Z¹⁰², more preferred is a C₁-C₆ alkyl group that may be substituted with Z¹⁰¹, a C₂-C₆ alkenyl group that may be substituted with Z¹⁰¹, or a phenyl group that may be substituted with Z¹⁰², still more preferred is a C₁-C₄ alkyl group that may be substituted with Z¹⁰¹ or the phenyl group that may be substituted with Z¹⁰², and further preferred is a methyl group or an ethyl group that may be substituted with Z¹⁰¹.

Besides, as R', preferred is the C₁-C₂₀ alkyl group that may be substituted with Z¹⁰³ or the C₆-C₂₀ aryl group that may be substituted with Z¹⁰⁴, more preferred is a C₁-C₁₀ alkyl group that may be substituted with Z¹⁰³ or a C₆-C₁₄ aryl group that may be substituted with Z¹⁰⁴, still more preferred is a C₁-C₆ alkyl group that may be substituted with Z¹⁰³ or a C₆-C₁₀ aryl group that may be substituted with Z¹⁰⁴, and further preferred is a C₁-C₄ alkyl group that may be substituted with Z¹⁰³ or a phenyl group that may be substituted with Z¹⁰⁴.

Note that the plurality of R groups may be identical or different; also, the plurality of R' groups may be identical or different.

As Z¹⁰¹, preferred is the halogen atom or the C₆-C₂₀ aryl group that may be substituted with Z¹⁰⁵, more preferred is a fluorine atom or a phenyl group that may be substituted with Z¹⁰⁵, and the most suitable is the absence (namely, non-substitution with Z¹⁰¹).

In addition, as Z¹⁰², preferred is the halogen atom or a C₆-C₂₀ alkyl group that may be substituted with Z¹⁰⁵, more preferred is a fluorine atom or a C₁-C₁₀ alkyl group that may be substituted with Z¹⁰⁵, and the most suitable is the absence (namely, non-substitution with Z¹⁰²).

On the other hand, as Z¹⁰³, preferred is the halogen atom, a phenyl group that may be substituted with Z¹⁰⁵, a furanyl group that may be substituted with Z¹⁰⁵, the epoxycyclohexyl group, the glycidoxy group, the methacryloxy group, the acryloxy group, the ureido group, the thiol group, the isocyanate group, the amino group, a phenylamino group that may be substituted with Z¹⁰⁵, or a diphenylamino group that may be substituted with Z¹⁰⁴, more preferred is the halogen atom, and still more preferred is a fluorine atom or the absence (namely, non-substitution with Z¹⁰³).

Besides, as Z¹⁰⁴, preferred is the halogen atom, the C₁-C₂₀ alkyl group that may be substituted with Z¹⁰⁵, the epoxycyclohexyl group, the glycidoxy group, the methacryloxy group, the acryloxy group, the ureido group, the thiol group, the isocyanate group, the amino group, a phenylamino group that may be substituted with Z¹⁰⁵, or a diphenylamino group that may be substituted with Z¹⁰⁵, more preferred is the halogen atom, and still more preferred is a fluorine atom or the absence (namely, non-substitution with Z¹⁰⁴).

Further, as Z¹⁰⁵, preferred is the halogen atom, and more preferred is a fluorine atom or the absence (namely, non-substitution with Z¹⁰⁵).

Specific examples of the organosilane compound which can be used in the present invention are given below, but they are not restrictive.

Specific examples of the dialkoxysilane compounds include dimethyldimethoxysilane, dimethyldiethoxysilane, methylethyldimethoxysilane, diethyldimethoxysilane, diethyldiethoxysilane, methylpropyldimethoxysilane, methylpropyldiethoxysilane, diisopropyldimethoxysilane, phenylmethyldimethoxysilane, vinylmethyldimethoxysilane,
3-glycidoxypropylmethyldimethoxysilane,
3-glycidoxypropylmethyldiethoxysilane,
3-(3,4-epoxycyclohexyl)ethylmethyldimethoxysilane,
3-methacryloxypropylmethyldimethoxysilane,
3-methacryloxypropylmethyldiethoxysilane,
3-mercaptopropylmethyldimethoxysilane,
3-aminopropylmethyldiethoxysilane,
N-(2-aminoethyl)aminopropylmethyldimethoxysilane, or
3,3,3-trifluoropropylmethyldimethoxysilane.

Specific examples of the trialkoxysilane compounds include methyltrimethoxysilane, methyltriethoxysilane, ethyltrimethoxysilane, ethyltriethoxysilane, propyltrimethoxysilane, propyltriethoxysilane, butyltrimethoxysilane, butyltriethoxysilane, pentyltrimethoxysilane, pentyltriethoxysilane, heptyltrimethoxysilane, heptyltriethoxysilane, octyltrimethoxysilane, octyltriethoxysilane, dodecyltrimethoxysilane, dodecyltriethoxysilane, hexadecyltrimethoxysilane, hexadecyltriethoxysilane, octadecyltrimethoxysilane, octadecyltriethoxysilane, phenyltrimethoxysilane, phenyltriethoxysilane, vinyltrimethoxysilane, vinyltriethoxysilane,
3-aminopropyltrimethoxysilane, 3-aminopropyltriethoxysilane, 3-glycidoxypropyltrimethoysilane,
3-glycidoxypropyltriethoxysilane,
3-methacryloxypropyltrimethoxysilane,
3-methacryloxypropyltriethoxysilane, triethoxy(4-(trifluoromethyl)phenyl)silane,
dodecyltriethoxysilane, 3,3,3-trifluoropropyltrimethoxysilane, (triethoxysilyl)cyclohexane,
perfluorooctylethyltriethoxysilane, triethoxyfluorosilane, tridecafluoro-1,1,2,2-tetrahydrooctyltriethoxysilane, pentafluorophenyltrimethoxysilane,
pentafluorophenyltriethoxysilane,
3-(heptafluoroisopropoxy)propyltriethoxysilane, heptadecafluoro-1,1,2,2-tetrahydrodecyltriethoxysilane. triethoxy-2-thienylsilane, or 3-(triethoxysilyl)furan.

Specific examples of the tetraalkoxysilane compounds include tetramethoxysilane, tetraethoxysilane, or tetrapropoxysilane.

Among these, preferred are
3,3,3-trifluoropropylmethyldimethoxysilane,
triethoxy(4-(trifluoromethyl)phenyl)silane,
3,3,3-trifluoropropyltrimethoxysilane,
perfluorooctylethyltriethoxysilane,
pentafluorophenyltrimethoxysilane, and
pentafluorophenyltriethoxysilane.

In the case where the charge-transporting varnish of the present invention contains the organosilane compound, the amount of the organosilane compound is normally approximately 0.1% to 50% by weight, based on the total amount of the charge-transporting substance or substances (where the dopant is contained, the total amount of the charge-transporting substance or substances and the dopant). From the viewpoints of restraining the charge-transporting properties of the thin film obtained from being lowered and enhancing the ability to inject holes into a layer which is laminated on the opposite side from an anode in such a manner as to contact a hole injection layer, such as a hole transport layer and a light-emitting layer, the amount of the organosilane compound is preferably approximately 0.5% to 40% by weight, more preferably approximately 0.8% to 30% by weight, and still more preferably approximately 1% to 20% by weight.

Note that the charge-transporting varnish of the present invention may contain a known charge-transporting substance or substances other than the oligoaniline derivative, in such a range as not to impair the effects of the present invention.

A method for preparing the charge-transporting varnish is not particularly limited. Examples of the method include a technique of dissolving the oligoaniline derivative of the present invention in a high-solvency solvent and adding a high-viscosity organic solvent to the resulting solution, and a technique of mixing a high-solvency solvent and a high-viscosity organic solvent and dissolving the oligoaniline derivative of the present invention in the resulting solvent mixture.

In the present invention, from the viewpoint of obtaining a higher-flatness thin film with good reproducibility, the charge-transporting varnish is desirably filtered by use of a submicro-order filter or the like after the charge-transporting substance and the dopant are dissolved in the organic solvent.

### [Charge-transporting Thin Film]

By applying the charge-transporting varnish of the present invention onto a substrate and baking the varnish, a charge-transporting thin film can be formed on the substrate.

Examples of the varnish-applying method include, but are not limited to, a dipping method, a spin coating method, a transfer printing method, a roll coating method, a brushing method, an ink jet method, a spraying method, or a slit coating method. It is preferable to control the viscosity and surface tension of the varnish, according to the coating method (varnish-applying method).

Besides, in the case of using the varnish of the present invention, a baking atmosphere is also not specifically restricted. Not only in an atmospheric air but also in an inert gas such as nitrogen or in vacuum, it is possible to obtain a thin film which has a uniform film forming surface and charge-transporting properties. From the viewpoint of obtaining a thin film having a high charge-transporting property with good reproducibility, however, the baking atmosphere is preferably the atmospheric air.

A baking temperature is appropriately set within the range of approximately 100°C to 260°C, taking into account the use of the thin film to be obtained or the degree of charge-transporting properties to be imparted to the thin film to be obtained. In the case where the thin film obtained is to be used as a hole injection layer of an organic EL element, the baking temperature is preferably approximately 140°C to 250°C, more preferably approximately 145°C to 240°C.

In addition, a baking time, which varies according to the baking temperature and cannot therefore be prescribed unconditionally, is normally approximately one minute to one hour.

Note that the baking may be conducted by changing the temperature at at least two stages, for the purpose of realizing a more uniform film-forming property or causing a reaction to progress on the substrate. The heating may well be carried out by use of an appropriate apparatus such as, for example, a hot plate or an oven.

The thickness of the charge-transporting thin film is not particularly limited. In the case where the charge-transporting thin film is used as a hole injection layer of an organic EL element, the thickness is preferably 5 nm to 200 nm. Examples of a method for varying the film thickness include a method in which the concentration of solid components in the varnish is varied, and a method in which the quantity of the solution on the substrate at the time of coating is varied.

While the charge-transporting thin film of the present invention can be preferably used as a hole injection layer in an organic EL element, it can also be used as a charge-transporting function layer such as a hole injection and transport layer.

### [Organic EL Elements]

The organic EL elements of the present invention each have a pair of electrodes, between which is provided the aforementioned charge-transporting thin film of the present invention.

Examples of a typical configuration of the organic EL element include, but are not limited to, the following (a) to (f). Note that in the following configurations, if necessary, an electron block layer or the like may be provided between a light-emitting layer and an anode, and a hole block layer or the like may be provided between the light-emitting layer and a cathode. In addition, a hole injection layer, a hole transport layer, or a hole injection and transport layer may function also as the electron block layer or the like, whereas an electron injection layer, an electron transport layer or an electron injection and transport layer may function also as the hole block layer or the like.
(a) Anode/hole injection layer/hole transport layer/light-emitting layer/electron transport layer/electron injection layer/cathode
(b) Anode/hole injection layer/hole transport layer/light-emitting layer/electron injection and transport layer/cathode
(c) Anode/hole injection and transport layer/light-emitting layer/electron transport layer/electron injection layer/cathode
(d) Anode/hole injection and transport layer/light-emitting layer/electron injection and transport layer/cathode
(e) Anode/hole injection layer/hole transport layer/light-emitting layer/cathode
(f) Anode/hole injection and transport layer/light-emitting layer/cathode

The "hole injection layer," the "hole transport layer," and the "hole injection and transport layer" are layers which are formed between the light-emitting layer and the anode and which have a function of transporting holes from the anode to the light-emitting layer. In the case where only one layer of a hole-transporting material is provided between the light-emitting layer and the anode, the hole-transporting material layer is the "hole injection and transport layer." In the case where at least two layers of hole-transporting materials are provided between the light-emitting layer and the anode, the hole-transporting material layer nearer to the anode is the "hole injection layer," and the other hole-transporting material layer or layers are each the "hole transport layer." Especially, as each of the hole injection layer and the hole injection and transport layer, there is used a thin film which is excellent not only in a property for accepting holes from the anode but also in a property for injecting the holes into the hole transport layer and the light-emitting layer, respectively.

The "electron injection layer," the "electron transport layer," and the "electron injection and transport layer" are layers which are formed between the light-emitting layer and the cathode and which have a function of transporting electrons from the cathode to the light-emitting layer. In the case where only one layer of an electron-transporting material is provided between the light-emitting layer and the cathode, the electron-transporting material layer is the "electron injection and transport layer." In the case where at least two layers of electron-transporting materials are provided between the light-emitting layer and the cathode, the electron-transporting material layer nearer to the cathode is the "electron injection layer" and the other electron-transporting material layer or layers are each the "electron transport layer."

The "light-emitting layer" is an organic layer which has a light-emitting function; in the case where a doping system is adopted, this layer contains a host material and a dopant material. In this case, the host material mainly has a function of promoting recombination of electrons and holes, and a function of confining excitons in the light-emitting layer. The dopant material has a function of causing the excitons obtained through recombination to emit light efficiently. In the case of a phosphorescent element, the host material mainly has a function of confining the excitons generated at the dopant in the light-emitting layer.

The charge-transporting thin film of the present invention can be preferably used as a hole injection layer, a hole transport layer, or a hole injection and transport layer, more preferably as the hole injection layer, in an organic EL element.

In the case of producing an organic EL element by use of the charge-transporting varnish of the present invention, examples of materials to be used and of a method to be used include, but are not limited to, the followings.

Electrode substrates to be used are preferably cleaned beforehand by preliminary washing with a detergent, an alcohol, or pure water. For example, an anode substrate is preferably subjected to a surface treatment such as an ultraviolet (UV) ozone treatment and an oxygen-plasma treatment. immediately before use. It is to be noted here, however, that such a surface treatment may not necessarily be carried out in the case where the anode material is composed mainly of an organic matter.

One example of the method of producing the organic EL element of the present invention, in the case where the thin film obtained from the charge-transporting varnish of the present invention is used as a hole injection layer, is as follows.

By the aforementioned method, the charge-transporting varnish of the present invention is applied to an anode substrate, followed by baking, to produce a hole injection layer on the electrode. A hole transport layer, a light-emitting layer, an electron transport layer, an electron injection layer, and a cathode are provided in this order over the hole injection layer. The hole transport layer, the light-emitting layer, the electron transport layer, and the electron injection layer may be formed by either of a vapor deposition method and a coating method (wet process), according to the characteristics of the materials used.

Examples of the anode material include transparent electrodes represented by indium tin oxide (ITO) and indium zinc oxide (IZO), and metal anodes composed of a metal represented by aluminum or an alloy thereof, which have preferably been subjected to a flattening treatment beforehand. Polythiophene derivatives and polyaniline derivatives which have high charge-transporting properties can also be used.

Note that examples of other metals for constituting the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and their alloys.

Examples of a material for forming the hole transport layer include hole-transporting low-molecular-weight materials such as (triphenylamine)dimer derivatives, [(triphenylamine)dimer]spirodimer, N,N'-bis(naphthalen-1-yl)-N,N'-bis(phenyl)-benzidine (α-NPD), N,N'-bis(naphthalen-2-yl)-N,N'-bis(phenyl)-benzidine, N,N'-bis(3-methylphenyl)-N,N'-bis(phenyl)-benzidine, N,N'-bis(3-methylphenyl)-N,N'-bis(phenyl)-9,9-spirobifluorene, N,N'-bis(naphthalene-1-yl)-N,N'-bis(phenyl)-9,9-spirobifluorene,
N,N'-bis(3-methylphenyl)-N,N'-bis(phenyl)-9,9-dimethyl-fluorene,
N,N'-bis(naphthalen-1-yl)-N,N'-bis(phenyl)-9,9-dimethyl-fluorene,
N,N'-bis(3-methylphenyl)-N,N'-bis(phenyl)-9,9-diphenyl-fluorene,
N,N'-bis(naphthalen-1-yl)-N,N'-bis(phenyl)-9,9-diphenyl-fluorene,
N,N'-bis(naphthalene-1-yl)-N,N'-bis(phenyl)-2,2'-dimethylbenzidine,
2,2',7,7'-tetrakis(N,N-diphenylamino)-9,9-spirobifluorene, 9,9-bis[4-(N,N-bis-biphenyl-4-yl-amino)phenyl]-9H-fluorene, 9,9-bis[4-(N,N-bis-naphthalen-2-yl-amino)phenyl]-9H-fluorene, 9,9-bis[4-(N-naphthalen-1-yl-N-phenyamino)-phenyl]-9H-fluorene, 2,2',7,7'-tetrakis[N-naphthalenyl(phenyl)-amino]-9,9-spirobifluorene,
N,N'-bis(phenanthren-9-yl)-N,N'-bis(phenyl)-benzidine, 2,2'-bis[N,N-bis(biphenyl-4-yl)amino]-9,9-spirobifluorene, 2,2'-bis(N,N-diphenylamino)-9,9-spirobifluorene, di-[4-(N,N-di(p-tolyl)amino)-phenyl]cyclohexane, 2,2',7,7'-tetra(N,N-di(p-tolyl)amino)-9,9-spirobifluorene, N,N,N',N'-tetra-naphthalen-2-yl-benzidine, N,N,N',N'-tetra-(3-methylphenyl)-3,3'-dimethylbenzidine. N,N'-di(naphthalenyl)-N,N'-di(naphthalen-2-yl)-benzidine, N,N,N',N'-tetra(naphthalenyl)-benzidine, N,N'-di(naphthalen-2-yl)-N,N'-diphenylbenzidine-1,4-diamine, N1,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine,
2,2'-bis(3-(N,N-di(p-tolyl)amino)phenyl)biphenyl, triarylamines such as
4,4',4"-tris[3-methylphenyl(phenyl)amino]triphenylamine (m-MTDATA), and
4,4',4"-tris[1-naphthyl(phenyl)amino]triphenylamine (1-TNATA), and
oligothiophenes such as
5,5"-bis-{4-[bis(4-methylpohenyl)amino]phenyl}-2,2':5',2"-terthiophene (BMA-3T).

Examples of a material for forming the light-emitting layer include tris(8-quinolinolato)aluminum(III) (Alq₃),
bis(8-quinolinolato)zinc(II) (Znq₂),
bis(2-methyl-8-quinolinolato)-4-(p-phenylphenolato)-aluminum(III) (BAlq),
4,4'-bis(2,2-diphenylvinyl)biphenyl,
9,10-di(naphthalen-2-yl)anthracene,
2-t-butyl-9,10-di(naphthalen-2-yl)anthracene, 2,7-bis[9,9-di(4-methylphenyl)-fluoren-2-yl]-9,9-di(4-methylphenyl)fluorene,
2-methyl-9,10-bis(naphthalen-2-yl)anthracene,
2-(9,9-spirobifluoren-2-yl)-9,9-spirobifluorene,
2,7-bis(9,9-spirobifluoren-2-yl)-9,9-spirobifluorene,
2-[9,9-di(4-methylphenyl)-fluoren-2-yl]-9,9-di(4-methylphenyl)fluorene,
2,2'-dipyrenyl-9,9-spirobifluorene,
1,3,5-tris(pyren-1-yl)benzene,
9,9-bis[4-(pyrenyl)phenyl]-9H-fluorene,
2,2'-bi(9,10-diphenylanthracene),
2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene,
3,10-di(naphthalen-2-yl)perylene,
tris[4-(pyrenyl)-phenyl]amine,
10,10'-di(biphenyl-4-yl)-9,9'-bianthracene,
N,N'-di(naphthalen-1-yl)-N,N'-diphenyl-[1,1':4',1":4",1"'-quaterphenyl]-4,4"'-diamine,
4,4'-di[10-(naphthalen-1-yl)anthracen-9-yl]biphenyl, dibenzo{[f,f']-4,4',7,7'-tetraphenyl}diindeno[1,2,3-cd:1',2',3'-Im]perylene,
1-(7-(9,9'-bianthracen-10-yl)-9.9-dimethyl-9H-fluoren-2-yl)pyrene,
1-(7-(9,9'-bianthracen-10-yl)-9,9-dihexyl-9H-fluoren-2-yl)pyrene,
1,3-bis(carbazol-9-yl)benzene,
1,3,5-tris(carbazol-9-yl)benzene.
4,4',4"-tris(carbazol-9-yl)triphenylamine,
4,4'-bis(carbazol-9-yl)biphenyl (CBP),
4,4'-bis(carbazol-9-yl)-2,2'-dimethylbiphenyl,
2,7-bis(carbazol-9-yl)-9,9-dimethylfluorene,
2,2',7,7'-tetrakis(carbazol-9-yl)-9,9-spirobifluorene,
2,7-bis(carbazol-9-yl)-9,9-di(p-tolyl)fluorene,
9,9-bis[4-(carbazol-9-yl)-phenyl]fluorene,
2,7-bis(carbazol-9-yl)-9,9-spirobifluorene,
1,4-bis(triphenylsilyl)benzene,
1,3-bis(triphenylsilyl)benzene,
bis(4-N,N-diethylamino-2-methylphenyl)-4-methylphenylmethane, 2,7-bis(carbazol-9-yl)-9,9-dioctylfluorene,
4,4"-di(triphenylsilyl)-p-terphenyl,
4,4'-di(triphenylsilyl)biphenyl,
9-(4-t-butylphenyl)-3,6-bis(triphenylsilyl)-9H-carbazole, 9-(4-t-butylphenyl)-3,6-ditrityl-9H-carbazole,
9-(4-t-butylphenyl)-3,6-bis(9-(4-methoxyphenyl)-9H-fluoren-9-yl)-9H-carbazole,
2,6-bis(3-(9H-carbazol-9-yl)phenyl)pyridine,
triphenyl(4-(9-phenyl-9H-fluoren-9-yl)phenyl)silane,
9,9-dimethyl-N,N-diphenyl-7-(4-(1-phenyl-1H-benzo[d]imidazol-2-yl)phenyl)-9H-fluoren-2-amine,
3,5-bis(3-(9H-carbazol-9-yl)phenyl)pyridine,
9,9-spirobifluoren-2-yl-diphenyl-phosphine oxide,
9,9'-(5-(triphenylsilyl)-1,3-phenylene)bis(9H-carbazole), 3-(2,7-bis(diphenylphosphoryl)-9-phenyl-9H-fluoren-9-yl)-9-phenyl-9H-carbazole,
4,4,8,8,12,12-hexa(p-tolyl)-4H-8H-12H-12C-azadibenzo-[cd,mn]pyrene,
4,7-di(9H-carbazol-9-yl)-1,10-phenanthroline, 2,2'-bis(4-(carbazol-9-yl)phenyl)biphenyl,
2,8-bis(diphenylphosphoryl)dibenzo[b,d]thiophene, bis(2-methylphenyl)diphenylsilane,
bis[3,5-di(9H-carbazol-9-yl)phenyl]diphenylsilane, 3,6-bis(carbazol-9-yl)-9-(2-ethyl-hexyl)-9H-carbazole, 3-(diphenylphosphoryl)-9-(4-(diphenylphosphoryl)phenyl)-9H-carbazole, or
3,5-bis[(3,5-diphenyl)phenyl]-9-phenylcarbazole. The light-emitting layer may be formed by co-evaporation of such a material with a light-emitting dopant.

Examples of the light-emitting dopant include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino[9,9a,lgh]coumarin, quinacridone, N,N'-dimethyl-quinacridone,
tris(2-phenylpyridine)iridium(III)(Ir(ppy)₃), bis(2-phenylpyridine)(acetylacetonate)iridium(III)
(Ir(ppy)₂(acac)),
tris[2-(p-tolyl)pyridine]iridium(III) (Ir(mppy)₃), 9,10-bis[N,N-di(p-tolyl)amino]anthracene,
9,10-bis[phenyl(m-tolyl)amino]anthracene,
bis[2-(2-hydroxyphenyl)benzothiazolato]zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine,
4,4'-bis(9-ethyl-3-carbazovinylene)-1,1'-biphenyl,
perylene, 2,5,8,11-tetra-t-butylperylene,
1,4-bis[2-(3-N-ethylcarbazolyl)vinyl]benzene,
4,4'-bis[4-(di-p-tolylamino)styryl]biphenyl,
4-(di-p-tolylamino)-4'-[(di-p-tolylamino)styryl]stilbene, bis[3,5-difluoro-2-(2-pyridyl)phenyl-(2-carboxypyridyl)]-iridium(III),
4,4'-bis[4-(diphenylamino)styryl]biphenyl,
bis(2,4-difluorophenylpyridinato)tetrakis(1-pyrazolyl)borate iridium(III),
N,N'-bis(naphthalen-2-yl)-N,N'-bis(phenyl)-tris(9,9-dimethylfluorenylene),
2,7-bis{2-[phenyl(m-tolyl)amino]-9,9-dimethyl-fluoren-7-yl}-9,9-dimethyl-fluorene,
N-(4-((E)-2-(6((E)-4-(diphenylamino)styryl)naphthalen-2-yl)vinyl)phenyl)-N-phenylbenzeneamine,
fac-iridium(III) tris(1-phenyl-3-methylbenzimidazolin-2-ylidene-C,C²),
mer-iridium(III) tris(1-phenyl-3-pmethylbenzimidazolin-2-ylidene-C,C²),
2,7-bis[4-(diphenylamino)styryl]-9,9-spirobifluorene, 6-methyl-2-(4-(9-(4-(6-methylbenzo[d]thiazol-2-yl)phenyl)-anthracen-10-yl)phenyl)benzo[d]thiazole,
1,4-di[4-(N,N-diphenyl)amino]styrylbenzene,
1,4-bis(4-(9H-carbazol-9-yl)styryl)benzene, (E)-6-(4-(diphenylamino)styryl)-N,N-diphenylnaphthalene-2-amine,
bis(2,4-difluorophenylpyridinato)(5-(pyridin-2-yl)-1H-tetrazolate)iridium(III),
bis(3-trifluoromethyl-5-(2-pyridyl)pyrazole)((2,4-difluorobenzyl)diphenylphosphinate)lridium(III),
bis(3-trifluoromethyl-5-(2-pyridyl)pyrazolate)(benzyldiphenylphosphinate)iridium(III),
bis(1-(2,4-difluorobenzyl)-3-methylbenzimidazolium)(3-(trifluoromethyl)-5-(2-pyridyl)-1,2,4-triazolate)iridium(III), bis(3-trifluoromethyl-5-(2-pyridyl)pyrazolate)(4',6'-difluorophenylpyridinate)iridium(III),
bis(4',6'-difluorophenylpyridinato)(3,5-bis(trifluoromethyl)-2-(2'-pyridyl)pyrrolate)iridium(III),
bis(4',6'-difluorophenylpyridinato)(3-(trifluoromethyl)-5-(2-pyridyl)-1,2,4-triazolate)iridium(III),
(Z)-6-mesityl-N-(6-mesitylquinolin-2(1H)-ylidene)quinoline-2-amine-BF₂, (E)-2-(2-(4-(dimethylamino)styryl)-6-metyl-4H-pyran-4-ylidene)malononitrile,
4-(dicyanomethylene)-2-methyl-6-julolidyl-9-enyl-4H-pyran, 4-(dicyanomethylene)-2-methyl-6-(1,1,7,7-tetramethyl-julolidyl-9-enyl)-4H-pyran,
4-(dicyanomethylene)-2-t-butyl-6-(1,1,7,7-tetramethyl-julolidin-4-yl-vinyl)-4H-pyran,
tris(dibenzoylmethane)phenanthroline europium(III), 5,6,11,12-tetraphenylnaphthacene,
bis(2-benzo[b]thiophen-2-yl-pyridine)(acetylacetonate)-iridium(III),
tris(1-phenylisoquinoline)iridium(III),
bis(1-phenylisoquinoline)(acetylacetonate)iridium(III), bis[1-(9,9-dimethyl-9H-fluoren-2-yl)-isoquinoline]-(acetylacetonate)iridium(III),
bis[2-(9,9-dimethyl-9H-fluoren-2-yl)quinoline]-(acetylacetonate)iridium(III),
tris[4,4'-di-t-butyl-(2,2')-bipyridine]-ruthenium(III)·bis(hexafluorophosphate),
tris(2-phenylquinoline)iridium(III),
bis(2-phenylquinoline)(acetylacetonate)iridium(III), 2,8-di-t-butyl-5,11-bis(4-t-butylphenyl)-6,12-diphenyl-tetracene,
bis(2-phenylbenzothiazolato)(acetylacetonate)iridium(III), 5,10,15,20-tetraphenyltetrabenzoporphyrin platinum, osmium(II) bis(3-trifluoromethyl-5-(2-pyridine)-pyrazolate)-dimethylphenylphosphine,
osmium(II) bis(3-(trifluoromethyl)-5-(4-t-butylpyridyl)-1,2,4-triazolate)diphenylmethylphosphine,
osmium(II) bis(3-(trifluoromethyl)-5-(2-pyridyl)-1,2,4-triazole)dimethylphenylphosphine,
osmium(II) bis(3-(trifluoromethyl)-5-(4-t-butylpyridyl)-1,2,4-triazolate)dimethylphenylphosphine,
bis[2-(4-n-hexylphenyl)quinoline](acetylacetonate)iridium(III), tris[2-(4-n-hexylphenyl)quinoline]iridium(III), tris[2-phenyl-4-methylquinoline]iridium(III),
bis(2-phenylquinoline)(2-(3-methylphenyl)pyridinate)-iridium(III),
bis(2-(9,9-diethyl-fluoren-2-yl)-1-phenyl-1H-benzo[d]-imidazolato)(acetylacetonate)iridium(III),
bis(2-phenylpyridine)(3-(pyridin-2-yl)-2H-chromen-2-onate)-iridum(III),
bis(2-phenylquinoline)(2,2,6,6-tetramethylheptane-3,5-dionate)iridium(III),
bis(phenylisoquinoline)(2,2,6,6-tetramethylheptane-3,5-dionate)iridium(III),
iridium(III)bis(4-phenylthieno[3,2-c]pyridinato-N,C²)-acetylacetonate,
(E)-2-(2-t-butyl-6-(2-(2,6,6-trimethyl-2,4,5,6-tetrahydro-1H-pyrolo[3,2,1-ij]quinolin-8-yl)vinyl)-4H-pyran-4-ylidene)-malononitrile,
bis(3-trifluoromethyl-5-(1-isoquinolyl)pyrazolate)(methyl-diphenylphosphine)ruthenium,
bis[(4-n-hexylphenyl)isoquinoline](acetylacetonate)-iridium(III),
platinum(II) octaethylporphine,
bis(2-methyldibenzo[f,h]quinoxaline)(acetylacetonate)-iridium(III), or
tris[(4-n-hexylphenyl)xisoquinoline]iridium(III).

Examples of a material for forming the electron transport layer include 8-hydroxyquinolinolato-lithium, 2,2',2"-(1,3,5-benzynetolyl)-tris(1-phenyl-1-H-benzimidazole), 2-(4-biphenyl)5-(4-t-butylphenyl)-1,3,4-oxadiazole, 2,9-dimethyl-4,7-diphenyl-1,10-phenanthroline, 4,7-diphenyl-1,10-phenanthroline,
bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine,
3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene,
1,3-bis[2-(4-t-butylphenyl)-1,3,4-oxadiazo-5-yl]benzene, tris(2,4,6-trimethyl-3-(bipyridin-3-yl)phenyl)borane, 1-methyl-2-(4-(naphthalen-2-yl)phenyl)-1H-imidazo-[4,5f][1,10]phenanthroline,
2-(naphthalene-2-yl)-4,7-diphenyl-1,10-phenanthroline, phenyl-dipyrenylphosphine oxide,
3,3',5,5'-tetra[(m-pyridyl)-phen-3-yl]biphenyl, 1,3,5-tris[(3-pyridyl)-phen-3-yl]benzene,
4,4'-bis(4,6-diphenyl-1,3,5-triazin-2-yl)biphenyl, 1,3-bis[3,5-di(pyridin-3-yl)phenyl]benzene,
bis(10-hydroxybenzo[h]quinolinato)beryllium,
diphenylbis(4-(pyridine-3-yl)phenyl)silane, or
3,5-di(pyren-1-yl)pyridine.

Examples of a material for forming the electron injection layer include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (StF₂), molybdenum trioxide (MoO₃), aluminum, lithium acetylacetonate (Li(acac)), lithium acetate, or lithium benzoate.

Examples of the cathode material include aluminum, magnesium-silver alloys, aluminum-lithium alloys, lithium, sodium, potassium, or cesium.

Other examples of the method of producing the organic EL element of the present invention, in the case where the thin film obtained from the charge-transporting varnish of the present invention is a hole injection layer, are as follows.

An organic EL element having the charge-transporting thin film formed from the charge-transporting varnish of the present invention can be produced by sequentially forming a hole transport layer and a light-emitting layer, in place of the operation of carrying out vapor deposition in vacuum to form the hole transport layer, the light-emitting layer, the electron transport layer, and the electron injection layer in the aforementioned method of producing the organic EL element. Specifically, the charge-transporting varnish of the present invention is applied onto an anode substrate to form a hole injection layer by the above-mentioned method, then a hole transport layer and a light-emitting layer are sequentially formed thereover, and, further, a cathode is vapor-deposited, to obtain the organic EL element.

As the cathode and anode materials to be used, the same materials as above-mentioned can be used, and they can be subjected to the same cleaning treatment and surface treatment as above-mentioned.

Examples of the method for forming the hole transport layer and the light-emitting layer include a method in which a hole-transporting polymer material or a light-emitting polymer material or an admixture thereof with a dopant is admixed with a solvent to dissolve or uniformly disperse the material (and the dopant, if used) in the solvent, and the resulting solution is applied individually onto the hole injection layer or the hole transport layer, followed by baking to form the desired film.

Examples of the hole-transporting polymer material include
poly[(9,9-dihexylfluorenyl-2,7-diyl)-co-(N,N'-bis{p-butylphenyl}-1,4-diaminophenylene)],
poly[(9,9-dioctylfluorenyl-2,7-diyl)-co-(N,N'-bis{p-butylphenyl}-1,1'-biphenylene-4,4-diamine)],
poly[(9,9-bis{1'-penten-5'-yl}fluorenyl-2,7-diyl)-co-(N,N'-bis{p-butylphenyl}-1,4-diaminophenylene)],
poly[N,N'-bis(4-butylphenyl)-N,N'-bis(phenyl)-benzidine]-endcapped with polysilsesquioxane, or
poly[(9,9-didioctylfluorenyl-2,7-diyl)-co-(4,4'-(N-(p-butylphenyl))diphenylamine)].

Examples of the light-emitting polymer material include polyfluorene derivatives such as poly(9,9-dialkylfluorenes) (PDAF), polyphenylenevinylene derivatives such as poly(2-methoxhy-5-(2'-ethylhexoxy)-1,4-phenylenevinylene) (MEH-PPV), polythiophene derivatives such as poly(3-alkylthiophenes) (PAT), or polyvinylcarbazole (PVCz).

Examples of the solvent include toluene, xylene, or chloroform. Examples of the method for dissolution or uniform dispersion include such a method as stirring, stirring with heating, and ultrasonic dispersion.

The coating method is not particularly limited. Examples of the coating method include an ink jet method, a spraying method, a dipping method, a spin coating method, a transfer printing method, a roll coating method, or a brushing method. Note that the coating is preferably conducted in an inert gas such as nitrogen and argon.

Examples of a baking method include a method of heating in an inert gas or in vacuum by use of an oven or a hot plate.

One example of the method for producing the organic EL element of the present invention, in the case where the thin film obtained from the charge-transporting varnish of the present invention is a hole injection and transport layer, is as follows.

A hole injection and transport layer is formed on an anode substrate, and a light-emitting layer, an electron transport layer, an electron injection layer, and a cathode are provided in this order over the hole injection and transport layer. Examples of the methods for forming the light-emitting layer, the electron transport layer, and the electron injection layer and specific examples of these layers include the same methods and layers as above-mentioned.

Examples of the anode material, the materials for forming the light-emitting layer, the light-emitting dopant, the electron transport layer, and the electron block layer, and the cathode material include the same materials as above-mentioned.

Note that, if necessary, a hole block layer, and/or an electron block layer may be provided between arbitrary ones of the electrodes and the layers. For example, the electron block layer may be formed from a material, examples of which include tris(phenylpyrazole)iridium or the like.

The materials constituting the anode, the cathode, and the layers formed between the anode and the cathode differ according to whether the element to be produced is of the bottom emission structure or of the top emission structure. Therefore, the materials are appropriately selected taking this point into account.

Normally, in an element of the bottom emission structure, a transparent anode is used on the substrate side, and light is taken out on the substrate side. On the other hand, in an element of the top emission structure, a reflective anode formed of a metal is used, and light is taken out on the side of a transparent electrode (cathode) present on the opposite side from the substrate. Therefore, as for the anode material, for example, a transparent anode of ITO or the like is used in producing an element of the bottom emission structure, whereas a reflective anode of Al/Nd or the like is used in producing an element of the top emission structure.

The organic EL element of the present invention may, if necessary, be sealed together with a desiccant or the like, according to an ordinary method, for the purpose of preventing the characteristics of the element from being degraded.

### EXAMPLES

The present invention will be described more specifically below by showing Examples and Comparative Examples, but the present invention is not limited to the following Examples. Note that the apparatuses which were used are as follows.
(1) ¹H-NMR measurement:
   JNM-ECP300 FT NMR SYSTEM, made by JEOL Ltd.
(2) Substrate cleaning:
   Substrate cleaning apparatus (vacuum plasma system), made by Choshu Industry Co., Ltd.
(3) Application of varnish:
   Spin coater MS-A100, made by Mikasa Co., Ltd.
(4) Film thickness measurement:
   Microfigure measuring instrument SURF-CORDER ET-4000, made by Kosaka Laboratory Ltd.
(5) Production of EL element:
   Multifunction deposition apparatus system C-E2L1G1, made by Choshu Industry Co., Ltd.
(6) Measurement of luminance, etc. of EL element:
   I-V-L measurement system, made by Tech World Inc.
(7) EL element lifetime measurement:
   Organic EL luminance evaluation system PEL-105S, made by EHC K.K.

### [1] Synthesis of Compound

### [Example 1-1] Synthesis of oligoaniline derivative 1

An oligoaniline derivative 1 was synthesized according to the following reaction formula.

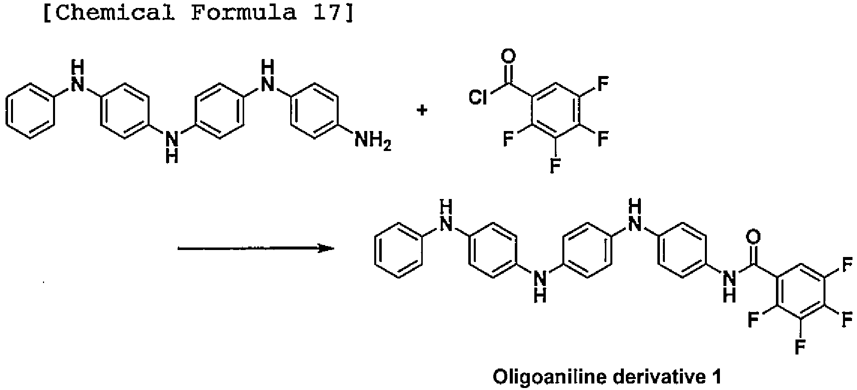

A flask was charged with 3.0 g of tetraaniline, 1.91 g of 2,3,4,5-tetrafluorobenzoyl chloride, and 60 g of N,N-dimethylacetamide, then the flask was flushed with nitrogen, and the contents were stirred at room temperature for one hour.

After the stirring was completed, 30 mL of a 5 mol/L aqueous sodium hydroxide solution was added, followed by stirring further for 30 minutes. Ethyl acetate and saturated aqueous common salt solution were mixed into the reaction mixture, and a liquid separation treatment was repeated (three times) until the pH became 7. The organic layer thus obtained was dried with sodium sulfate, followed by vacuum concentration. To the resulting concentrated liquid, was added 15 mL of THF. The solution thus obtained was added dropwise to 210 mL of isopropyl alcohol, and the resulting slurry was stirred at room temperature for 30 minutes.

Finally, the slurry solution was filtered, and the filter cake obtained was dried, to obtain the desired oligoaniline derivative 1 (yield: 2.94 g). The results of ¹H-NMR measurement are given below.
¹H-NMR (400MHz, DMSO-d6) δ[ppm]: 10.35(s, 1H), 7.83(s, 1H), 7.79-7.68(m, 3H), 7.49(d, J=8.0Hz, 2H), 7.15(t, J=8.0Hz, 2H), 7.01-6.90(m, 12H), 6.68(t, J=8.0Hz, 1H)

### [Example 1-2] Synthesis of oligoaniline derivative 2

An oligoaniline derivative 2 was synthesized according to the following reaction formula.

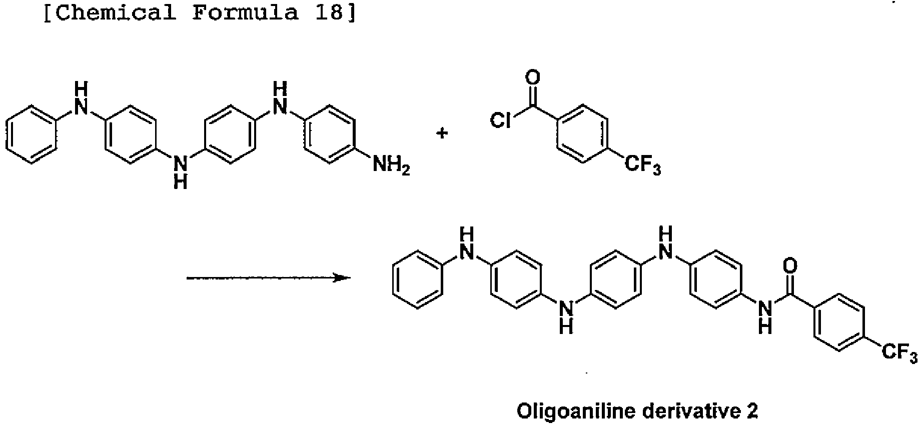

A flask was charged with 1.83 g of tetraaniline, 1.91 g of 4-(trifluoromethyl)benzoyl chloride, and 18 g of N,N-dimethylacetamide, then the flask was flushed with nitrogen, and the contents were stirred at room temperature for one hour.

After the stirring was completed, 18 mL of a 5 mol/L aqueous sodium hydroxide solution was added, followed by stirring further for 30 minutes. Ethyl acetate and saturated aqueous common salt solution were added to the reaction mixture, and a liquid separation treatment was repeated (three times) until the pH become 7. The organic layer thus obtained was dried with sodium sulfate, followed by vacuum concentration. To the resulting concentrated liquid, was added 9 mL of THF. The solution thus obtained was added dropwise to 128 mL of isopropyl alcohol, and the resulting slurry was stirred at room temperature for 30 minutes.

Finally, the slurry solution was filtered, and the resulting filter cake was dried, to obtain the desired oligoaniline derivative 2 (yield: 2.04 g). The results of ¹H-NMR measurement are given below.
¹H-NMR (400MHz, DMSO-d6) δ[ppm]: 10.27(s, 1H), 8.14(d, J=8.0Hz, 2H), 7.90(d, J=8.4Hz, 2H), 7.80(d, J=10.0Hz, 2H), 7.68(s, 1H), 7.57(d, J=8.8Hz, 2H), 7.15(t, J=8.0Hz, 3H), 7.01-6.90(m, 3H), 7.49(d, J=8.0Hz, 2H), 7.15(t, J=8.0Hz, 2H), 7.01-6.90(m, 12H), 6.68(t, J=7.2Hz, 1H)

### [Synthesis Example 1] Synthesis of arylsulfonic acid 1

An arylsulfonic acid 1 was synthesized according to the following reaction formula.

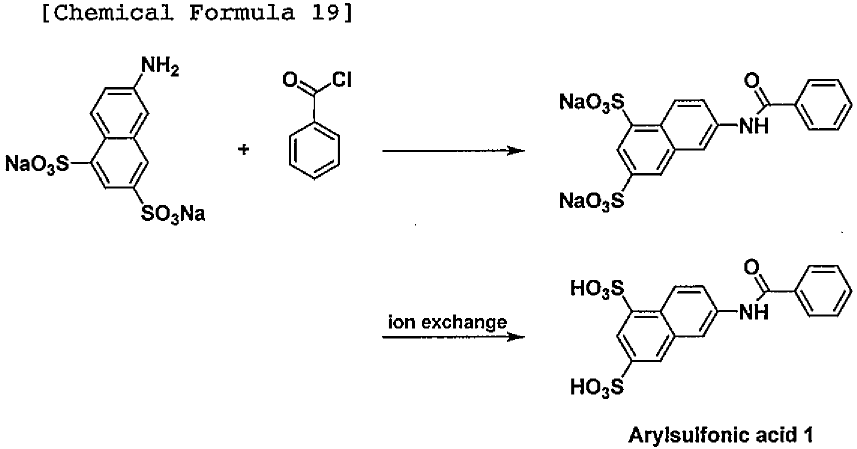

A flask flushed with nitrogen was charged with 10.0 g of disodium 6-amino-1,3-naphthalenedisulfonate hydrate (made by Sugai Chemical Industry Co., Ltd.) and 60 g of N,N-dimethylacetamide, followed by stirring at 50°C for 10 minutes. Then, 4.32 g of benzoyl chloride (made by Tokyo Chemical Industry Co., Ltd.) was added to the flask, and the contents were stirred with heating at 50°C for 15 minutes.

After the stirring was completed, the reaction mixture was let cool, the solvent was distilled off under vacuum, and the resulting residue was mixed well with 40 mL of methanol.

Next, the mixture thus obtained was filtered, the filtrate was slowly added dropwise to an isopropanol-hexane mixed solvent (volume ratio, 3:1), and the solid matter precipitated was collected by filtration. The solid matter (filter cake) thus collected was dried well in vacuum, was then dissolved in 20 mL of pure water, and the resulting aqueous solution was passed through a column packed with a cation exchange resin DOWEX 650C (H type; approximately 100 mL; distillation solvent: water), whereby cation exchange and removal of impurities were conducted.

Finally, water was distilled off from the aqueous solution, and the resulting solid matter was dried well, to obtain the desired arylsulfonic acid 1 (yield: 10.9 g). The results of ¹H-NMR measurement are given below.
¹H-NMR (400MHz, DMSO-d6) δ[ppm]: 10.52(S, 1H), 8.76 (d, J=9.2Hz, 1H), 8.44(S, 1H), 8.16(S, 1H), 8.03(t, J=7.6Hz, 3H), 7.89(dd, J=9.6, 2.0Hz, 1H), 7.64-7.55(m, 3H)

### [2] Preparation of Charge-transporting Varnish

### [Example 2-1]

In a nitrogen atmosphere, 0.173 g of the oligoaniline derivative 1 and 0.195 g of the arylsulfonic acid 1 as a dopant were dissolved in 6 g of 1,3-dimethyl-2-imidazolidinone. To the resulting solution, were added 9 g of cyclohexanol and 3 g of propylene glycol, followed by stirring, to prepare a charge-transporting varnish.

### [Example 2-2]

In a nitrogen atmosphere, 0.218 g of the oligoaniline derivative 1 and 0.272 g of an arylsulfonic acid 2 represented by the following formula, as a dopant, were dissolved in 8 g of 1,3-dimethyl-2-imidazolidinone. To the resulting solution, were added 12 g of cyclohexanol and 4 g of propylene glycol, followed by stirring, to prepare a charge-transporting varnish. Note that the arylsulfonic acid 2 was synthesized according to WO 2006/025342.

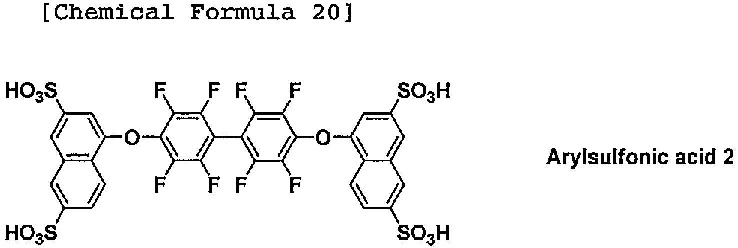

### [Example 2-3]

In a nitrogen atmosphere, 0.089 g of the oligoaniline derivative 1 and 0.111 g of the arylsulfonic acid 2 were dissolved in 3.3 g of 1,3-dimethyl-2-imidazolidinone. To the resulting solution, were added 4.9 g of cyclohexanol and 1.6 g of propylene glycol, followed by stirring, and then 0.007 g of 3,3,3-trifluoropropyltrimethoxysilane and 0.013 g of phenyltrimethoxysilane were added, followed by stirring, to prepare a charge-transporting varnish.

### [3] Production and Characteristics Evaluation of Organic EL Element

### [Example 3-1]

The varnish obtained in Example 2-1 was applied to an ITO substrate by use of a spin coater, after which the resulting coating was dried at 80°C for five minutes, and was further baked at 230°C in the atmospheric air for 10 minutes, to form a 30 nm-thick uniform thin film on the ITO substrate. As the ITO substrate, a 25 mm x 25 mm x 0.7 (t) glass substrate provided on its surface with a pattern of indium tin oxide (ITO) in a thickness of 150 nm was used, after impurities on the surface of the ITO substrate were removed by an O₂ plasma cleaning apparatus (150 W, 30 seconds).

Next, thin films of α-NPD, Alq₃, lithium fluoride, and aluminum were sequentially layered over the ITO substrate (having been provided thereon with the thin film) by use of a vapor deposition apparatus (vacuum degree 1.0×10⁻⁵ Pa), to obtain an organic EL element. In this case, the vapor deposition rate was 0.2 nm/second for α-NPD, Alq₃, and aluminum, and 0.02 nm/second for lithium fluoride. Each of the thicknesses of the thin films was 30 nm, 40 nm, 0.5 nm, and 120 nm.

Note that for preventing characteristics of the organic EL element from being deteriorated under the influences of oxygen and moisture in air, the organic EL element was sealed by use of sealing substrates, before evaluation of the characteristics. The sealing was conducted according to the following procedure.

In a nitrogen atmosphere having an oxygen concentration of up to 2 ppm and a dew point of up to -85°C, the organic EL element was placed between the sealing substrates, and the sealing substrates were laminated together using an adhesive (MORESCO MOISTURE CUT WB90US(P), made by Moresco Corporation). In this case, a desiccant (HD-071010W-40, made by Dynic Corporation) was placed together with the organic EL element inside the sealing substrates. The adhesive was cured by irradiating the laminated sealing substrates with UV light (wavelength, 365 nm; dosage, 6,000 mJ/cm²), and annealing at 80° C for one hour.

### [Examples 3-2 and 3-3]

Organic EL elements were produced by the same manner as in Example 3-1, except that the varnishes obtained in Examples 2-2 and 2-3 were used in place of the varnish obtained in Example 2-1.

These elements were put to measurement of current density, luminance, current efficiency, and half-life (initial luminance, 5,000 cd/m²) at a driving voltage of 5 V. The results are set forth in Table 4. Note that the area as size of the light-emitting surface of each of the elements is 2 mm x 2 mm.

**[Table 4]**

| | Current density (mA/cm²) | Luminance (cd/m²) | Current efficiency (cd/A) | Half-life (h) |
|---|---|---|---|---|
| Example 3-1 | 80.9 | 2,510 | 3.1 | 395 |
| Example 3-2 | 111.6 | 3,157 | 2.8 | 413 |
| Example 3-3 | 110.4 | 3,160 | 2.9 | 420 |

As depicted in Table 4, it was seen that by use of the charge-transporting varnish containing the oligoaniline derivative and the charge-transporting substance of the present invention, a charge-transporting thin film which is preferable for use as a hole injection layer and which enables realization of an organic EL element having a high luminance can be obtained. In addition, such elements were found to be excellent in durability.

## Claims

1. An oligoaniline derivative represented by formula (1): 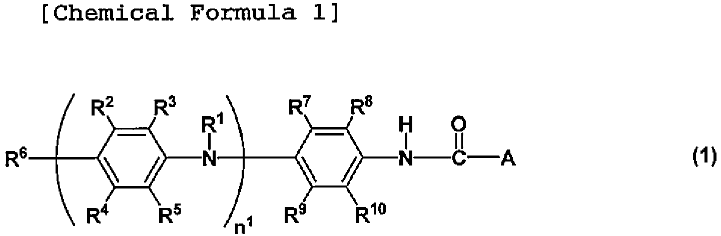 (In the formula, R¹ represents a hydrogen atom or a C₁-C₂₀ alkyl group that may be substituted with Z, Z represents a halogen atom, a nitro group, a cyano group, an aldehyde group, a hydroxyl group, a thiol group, a sulfonic acid group, a carboxylic acid group, a C₆-C₂₀ aryl group that may be substituted with Z', or a C₂-C₂₀ heteroaryl group that may be substituted with Z¹, and Z' represents a halogen atom, a nitro group, a cyano group, an aldehyde group, a hydroxyl group, a thiol group, a sulfonic acid group, or a carboxylic acid group;
R² to R¹⁰ each independently represent a hydrogen atom, a halogen atom, a nitro group, a cyano group, or a C₁-C₂₀ alkyl group, a C₂-C₂₀ alkenyl group, a C₂-C₂₀ alkynyl group, a C₆-C₂₀ aryl group, or a C₂-C₂₀ heteroaryl group that may be substituted with a halogen atom;
A represents
a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group that may be substituted with a cyano group, a chlorine atom, a bromine atom, an iodine atom, a nitro group, or a C₁-C₂₀ fluoroalkoxy group,
a C₆-C₂₀ fluoroaryl group that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group,
a C₆-C₂₀ aryl group that is substituted with C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₁-C₂₀ fluoroalkynyl group and that may be substituted with a cyano group, a halogen atom, or a C₁-C₂₀ fluoroalkoxy group,
a C₇-C₂₀ fluoroaralkyl group that may be substituted with a cyano group, a chlorine atom, a bromine atom, an iodine atom, a nitro group, a C₁-C₂₀ fluoroalkoxy group, a C₁-C₂₀ fluoroalkyl group, a C₃-C₂₀ fluorocycloalkyl group, a C₁-C₂₀ fluorobicycloalkyl group, a C₂-C₂₀ fluoroalkenyl group, or a C₂-C₂₀ fluoroalkynyl group, or
a C₇-C₂₀ aralkyl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group; and
letter n¹ represents an integer of 1 to 20.)

2. The oligoaniline derivative according to claim 1, wherein A is a C₁-C₂₀ fluoroalkyl group that may be substituted with a cyano group, a chlorine atom, a bromine atom, an iodine atom, a nitro group, or a C₁-C₂₀ fluoroalkoxy group, a C₆-C₂₀ fluoroaryl group that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group, or a C₆-C₂₀ aryl group that is substituted with a C₁-C₂₀ fluoroalkyl group, a C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group.

3. The aniline derivative according to claim 2, wherein A is a phenyl group that is substituted with at least three fluorine atoms and that may be substituted with a cyano group, a chlorine atom, a bromine atom, an iodine atom, a nitro group, a C₁-C₂₀ alkyl group, a C₁-C₂₀ fluoroalkyl group, or a C₁-C₂₀ fluoroalkoxy group, or a phenyl group that is substituted with C₃-C₂₀ fluorocycloalkyl group, a C₄-C₂₀ fluorobicycloalkyl group, a C₂-C₂₀ fluoroalkenyl group, or a C₂-C₂₀ fluoroalkynyl group and that may be substituted with a cyano group, a halogen atom, or a C₁-C₂₀ fluoroalkoxy group.

4. The oligoaniline derivative according to any one of claims 1 to 3, wherein R¹ is a hydrogen atom.

5. The oligoaniline derivative according to any one of claims 1 to 4, wherein R² to R¹⁰ are each a hydrogen atom.

6. The oligoaniline derivative according to any one of claims 1 to 5, wherein letter n¹ is 2 to 10.

7. A charge-transporting substance consisting of the oligoaniline derivative according to any one of claims 1 to 6.

8. A charge-transporting varnish containing the charge-transporting substance according to claim 7 and an organic solvent.

9. The charge-transporting varnish according to claim 8, wherein the varnish further contains a dopant.

10. A charge-transporting thin film produced by use of the charge-transporting varnish according to claim 8 or 9.

11. An electronic device comprising the charge-transporting thin film according to claim 10.

12. An organic electroluminescent element comprising the charge-transporting thin film according to claim 10.

13. A method of producing the oligoaniline derivative according to claim 1, the method comprising reacting an amine compound represented by formula (2) with a fluorine-containing acid halide represented by formula (3). 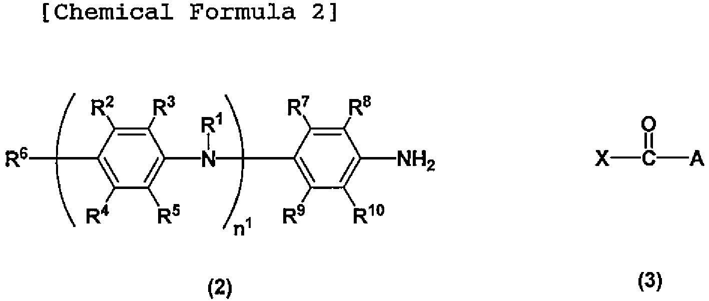 (In the formulas, R¹ to R¹⁰, A, and letter n¹ are the same as defined above, and X represents a halogen atom.)
